# EUROPEAN PATENT SPECIFICATION

(11) **EP 3 755 375 B1**
(45) Date of publication and mention of the grant of the patent: **05.03.2025**
(21) Application number: 19712287.2
(22) Date of filing: 22.02.2019
(51) Int. Cl.: A61K 47/36, A61K 47/38, A61K 31/335, A61K 31/58

(54) **TREATMENT OF ALLERGIC RHINITIS IN PEDIATRIC SUBJECTS USING A COMBINATION OF MOMETASONE AND OLOPATADINE**
BEHANDLUNG VON ALLERGISCHER RHINITIS BEI PÄDIATRISCHEN PATIENTEN UNTER VERWENDUNG EINER KOMBINATION VON MOMETASON UND OLOPATADIN
TRAITEMENT DE LA RHINITE ALLERGIQUE CHEZ DES SUJETS PÉDIATRIQUES À L'AIDE D'UNE COMBINAISON DE MOMÉTASONE ET D'OLOPATADINE

(30) Priority: 23.02.2018 US 201815903597
(43) Date of publication of application: 30.12.2020
(73) Proprietor: Glenmark Specialty S.A., 2300 La Chaux-de-Fonds (CH)
(72) Inventor: KHAIRATKAR-JOSHI, Neelima, Pachpakhadi, Thane 400602 (IN); KULKARNI, Abhay, Navi Mumbai 400709 (IN); WALE, Dinesh Pradeep, District-Osmanabad, Maharashtra 413601 (IN); BHOSALE, Vikram M., Siwree, Mumbai 400015 (IN); AGARWAL, Piyush, Malad (west), Mumbai 400064 (IN); KEOHANE, Patrick, London SW1P 1PD (GB); TANTRY, Sudeesh K., Jamison, PA 18929-1772 (US); OH, Chad, San Francisco, CA 94107-5492 (US)
(74) Representative: HGF
(86) International application number: PCT/IB2019/051465
(87) International publication number: WO 2019/162902

(56) References cited:
- US-A1- 2015 099 725
- US-A1- 2016 287 612
- GLENMARK PHARMACEUTICALS: "Glenmark Pharmaceuticals Reports Positive Results from a Phase 3 Trial of GSP 301,", 29 March 2017 (2017-03-29), XP055444412, Retrieved from the Internet <URL:https://www.prnewswire.com/news-releases/glenmark-pharmaceuticals-reports-positive-results-from-a-phase-3-trial-of-gsp-301-mometasoneolopatadine-fixed-dose-combination-nasal-spray-in-seasonal-allergic-rhinitis-300431125.html> [retrieved on 20180125]
- ANONYMOUS: "NCT02870205 on 2017_05_03: ClinicalTrials.gov Archive", 3 May 2017 (2017-05-03), XP055444415, Retrieved from the Internet <URL:https://clinicaltrials.gov/archive/NCT02870205/2017_05_03> [retrieved on 20180125]
- ANONYMOUS: "NCT02631551 on 2017_05_18: ClinicalTrials.gov Archive", 18 May 2017 (2017-05-18), XP055444418, Retrieved from the Internet <URL:https://clinicaltrials.gov/archive/NCT02631551/2017_05_18> [retrieved on 20180125]
- KALINER ET AL: "Azelastine and olopatadine in the treatment of allergic rhinitis", ANNALS OF ALLERGY, ASTHMA & IMMUNOLOGY, ARLINGTON HEIGHTS, IL, US, vol. 103, no. 5, 1 November 2009 (2009-11-01), pages 373 - 380, XP026960456, ISSN: 1081-1206, [retrieved on 20091101]
- ANONYMOUS: "To Study GSP 301 in Patients With Seasonal Allergic Rhinitis - Study Results - ClinicalTrials.gov", 28 June 2017 (2017-06-28), XP055445229, Retrieved from the Internet <URL:https://www.clinicaltrials.gov/ct2/show/results/NCT02318303?term=NCT02318303&rank=1 =X4370156#othr> [retrieved on 20180126]

## Description

This patent application is a continuation-in-part application of U.S. Patent Application No. 15/903,597, filed February 23, 2018.

### TECHNICAL FIELD OF THE INVENTION

The present patent application relates to a method of treating allergic rhinitis in a pediatric subject by administering a combination of mometasone or its salt and olopatadine or its salt as defined by the appended claims.

### BACKGROUND OF THE INVENTION

Allergic rhinitis is a medical term for inflammation and irritation of the mucous membrane inside the nose. It generally occurs when an allergen such as pollen, dust, or animal dander (particles of shed skin and hair) is inhaled by an individual with a sensitized immune system. Allergic rhinitis may cause additional symptoms such as rhinorrhea (excess nasal secretion), sneezing, nasal itching, nasal congestion and obstruction, coughing, headache, fatigue and malaise. Symptoms may vary in severity between individuals.

Many treatment options are available for treating allergic rhinitis such as, for example, antihistamines (e.g., cetirizine and loratadine), steroids (e.g., triamcinolone), decongestants, and leukotriene receptor antagonists (e.g., montelukast). These treatments are generally administered orally or nasally and some are associated with unpleasant taste and smell (e.g., Dymista^{®} nasal spray, which is a combination of azelastine and fluticasone propionate).

Olopatadine hydrochloride, an antihistamine, is chemically described as (Z)-11-[3-(dimethylamino)propylidene]-6,11-dihydrodibenz[b,e]oxepin-2-acetic acid hydrochloride, and is disclosed in U.S. Patent Nos. 4,871,865 and 4,923,892. It is commercially available in the United States as PATANASE^{®} Nasal Spray, which contains 0.6% w/v olopatadine (base) in a non-sterile aqueous solution. It is indicated for the relief of the symptoms of seasonal allergic rhinitis (SAR) in adults and children 6 years of age and older. PATANASE^{®} was found to have an onset of action of 30 minutes after dosing in the environmental exposure unit (Ref: USFDA approved label for Patanase).

Mometasone furoate is a glucocorticosteroid used topically to reduce inflammation of the skin or in the airways. Mometasone furoate monohydrate is commercially available in the United States as NASONEX^{®}, a nasal spray indicated for (i) the treatment of nasal symptoms of allergic rhinitis in patients ≥2 years of age, (ii) the treatment of nasal congestion associated with seasonal allergic rhinitis in patients ≥2 years of age, (iii) the prophylaxis of seasonal allergic rhinitis in patients ≥ 12 years of age, and (iv) the treatment of nasal polyps in patients ≥18 years of age. It is available as 50 mcg in a metered-dose, manual pump spray unit containing an aqueous suspension of mometasone furoate monohydrate equivalent to 0.05% w/w mometasone furoate (calculated on the anhydrous basis).

International Publication No. WO 2011/141929 discloses an aqueous nasal spray solution comprising fluticasone and olopatadine.

U.S. Patent No. 6,127,353 discloses a pharmaceutical composition of mometasone furoate monohydrate.

U.S. Patent Nos. 7,977,376 and 8,399,508 disclose a topical formulation of olopatadine hydrochloride.

International Publication No. WO 2011/008923 discloses a nasal spray regimen of olopatadine for children.

International Publication No. WO 1995/020393 discloses the use of mometasone furoate for treating airway passage and lung diseases.

International Publication No. WO 2010/025236 discloses a combination of a nasal steroid and a nasal antihistamine for the treatment of viral upper respiratory tract infections, upper respiratory infections, and common colds. WO2015/049665 discloses a combination of Monometasone furoate and olopatadine hydrochloride in treating allergic rhinitis.

### SUMMARY OF THE INVENTION

The present invention relates to a fixed dose combination of mometasone or its salt and olopatadine or its salt and its use for the treatment of rhinitis in a pediatric subject in need thereof. The inventors have surprisingly found that mometasone furoate and olopatadine hydrochloride act synergistically in the treatment of allergic rhinitis and the combination is more effective and provides better therapeutic value in pediatric subjects than treatment with either active ingredient alone. The fixed dose combination of mometasone furoate and olopatadine hydrochloride administered as one spray, twice daily, where each spray provides 25 mcg mometasone furoate and 665 mcg olopatadine hydrochloride, results in a significant improvement in the quality of life for pediatric subjects, including a significant reduction in symptoms of allergic rhinitis.

The inventors have also surprisingly found that nasal administration of a pharmaceutical composition of mometasone or a salt thereof (such as mometasone furoate) and olopatadine or a salt thereof (such as olopatadine hydrochloride) provides a faster onset of action of relief of symptoms associated with allergic rhinitis, such as seasonal allergic rhinitis or perennial allergic rhinitis, when compared to olopatadine hydrochloride monotherapy or mometasone furoate monotherapy. In particular, the pharmaceutical composition provides faster relief of nasal symptoms, such as nasal congestion, rhinorrhea, itching and sneezing. The pharmaceutical composition may also provide a faster onset of action of ocular symptoms, such as ocular itching, tearing/watery eyes and ocular redness. The onset of action may be less than 30 minutes, such as within about 15 minutes, such as within about 10 minutes.

Described is a method of treating allergic rhinitis in a pediatric human subject in need thereof comprising nasally administering to the subject an effective amount of a fixed-dose pharmaceutical composition comprising mometasone or its salt and olopatadine or its salt. Preferably, the composition is nasally administered as 1 spray per nostril of the subject twice daily. Each spray preferably comprises mometasone or its salt and olopatadine or its salt in a weight ratio of about 1:5 to about 1:60, such as in a weight ratio of from about 1:12 to about 1:53, from about 1:13.3 to about 1:50, or from about 1:18 to about 1:40 (based on the equivalent weight of olopatadine free base). In one embodiment, each spray provides about 665 mcg olopatadine hydrochloride and about 25 mcg mometasone furoate. In one embodiment, the fixed-dose pharmaceutical composition is a suspension wherein the mometasone or its salt is present in particulate form and the olopatadine or its salt is present in dissolved form.

Described is a method of treating allergic rhinitis in a pediatric human subject in need thereof comprising nasally administering to the subject an effective amount of a fixed-dose pharmaceutical composition comprising mometasone furoate monohydrate and olopatadine hydrochloride. In one preferred embodiment, the composition is nasally administered as 1 spray per nostril of the subject twice daily. Each spray of the pharmaceutical composition may comprise olopatadine hydrochloride equivalent to about 300 mcg, about 450 mcg, about 600 mcg, about 750 mcg, or about 900 mcg of olopatadine, and about 12.5 mcg, about 25 mcg, about 37.5 mcg, about 50 mcg, or about 62.5 mcg of mometasone furoate. In one embodiment, each spray comprises olopatadine hydrochloride equivalent to about 600 mcg of olopatadine and about 25 mcg of mometasone furoate.

Allergic rhinitis in the context of present invention includes, but is not limited to, inflammation and irritation of the mucous membrane inside the nose, and nasal and/or non-nasal symptoms associated therewith. It includes, for example, persistent allergic rhinitis, perennial allergic rhinitis, seasonal allergic rhinitis, chronic rhinitis, rhinitis medicamentosa, vasomotor rhinitis, infective rhinitis, autonomic rhinitis, hormonal rhinitis, drug-induced rhinitis, atrophic rhinitis, and gustatory rhinitis. Preferably, the allergic rhinitis is selected from perennial allergic rhinitis, persistent allergic rhinitis, seasonal allergic rhinitis, and nasal and/or non-nasal symptoms associated therewith.

In the context of the present invention, symptoms associated with rhinitis includes rhinorrhea, nasal congestion, nasal itching, sneezing, itching/burning eyes, tearing/watering eyes, redness of eyes, itching of ears or palate, coughing, ocular pruritus, excess lacrimation, headache, fatigue and malaise.

Described is a method of treating allergic rhinitis in a pediatric human subject in need thereof, the method comprising nasally administering to the subject an effective amount of a fixed-dose pharmaceutical composition comprising mometasone or its salt and olopatadine or its salt in a weight ratio of about 1:5 to about 1:60 or from about 1:12 to about 1:53, wherein (i) the composition is nasally administered as 1 spray per nostril, twice daily, and (ii) each spray comprises olopatadine hydrochloride equivalent to about 600 mcg of olopatadine and about 25 mcg of mometasone furoate. In one embodiment, the composition is administered for about 1 week. In another aspect of the embodiment, the composition is administered for about 2 weeks.

In one embodiment, the total nasal symptoms score (TNSS) of the human subject is reduced by at least 40 %, preferably by at least 50 % from baseline after 1 or 2 weeks treatment. In another embodiment, the total ocular symptom score (TOSS) of the human subject is reduced by at least 30 %, preferably by at least 40 % from baseline after 1 or 2 weeks treatment. In an aspect of the invention, the total nasal symptoms score (TNSS) and the total ocular symptom score (TOSS) can be observed as instantaneous or reflective or both.

Yet another embodiment is a method of treating symptoms associated with allergic rhinitis in a pediatric human subject in need thereof comprising nasally administering twice daily, one spray per nostril of a fixed-dose pharmaceutical composition comprising mometasone or its salt (e.g., mometasone furoate) and olopatadine its salt (e.g., olopatadine hydrochloride). Each spray may comprise mometasone or its salt and olopatadine or its salt in a weight ratio of about 1:5 to about 1:60 (such as a weight ratio of about 1:12 to about 1:53, about 1:13.3 to about 1:50, or from about 1:18 to about 1:40) (based on the equivalent weight of olopatadine free base) (for example, each spray comprises about 12.5 mcg, about 25 mcg, about 37.5 mcg, about 50 mcg, or about 62.5 mcg of mometasone or its salt (such as about 50 mcg mometasone furoate) and olopatadine hydrochloride equivalent to about 300 mcg, about 450 mcg, about 600 mcg, about 750 mcg, or about 900 mcg of olopatadine (such as about 665 mcg olopatadine hydrochloride)). In one embodiment, each spray provides about 665 mcg olopatadine hydrochloride and about 25 mcg mometasone furoate. The administration may provide relief from one or more symptoms of allergic rhinitis (such as nasal symptoms or ocular symptoms) faster (e.g., an onset of action in less than 30 minutes, such as within about 15 minutes, such as within 10 minutes) than nasal administration of the mometasone or its salt or the olopatadine or its salt alone. In another embodiment, the administration may provide relief from one or more symptoms of allergic rhinitis (such as nasal symptoms) in a subject exposed to an environmental exposure chamber (EEC) (such as one with ragweed pollen at a concentration of 3500±500 particles/m³ for 6 hours) faster (e.g., an onset of action in less than 15 minutes, such as within about 10 minutes) than nasal administration of the mometasone or its salt or the olopatadine or its salt alone.

Described is a method for providing faster onset of relief of symptoms associated with allergic rhinitis in a pediatric human subject in need thereof comprising nasally administering twice daily, one spray per nostril of a fixed-dose pharmaceutical composition comprising mometasone or its salt (e.g., mometasone furoate) and olopatadine its salt (e.g., olopatadine hydrochloride). This method may provide faster onset of relief of one or more symptoms compared to administration of the mometasone or its salt alone or olopatadine or its salt alone. Each spray may comprise mometasone or its salt and olopatadine or its salt in a weight ratio of about 1:5 to about 1:60 (such as a weight ratio of about 1:12 to about 1:53, about 1:13.3 to about 1:50, or from about 1:18 to about 1:40) (based on the equivalent weight of olopatadine free base) (for example, each spray comprises about 12.5 mcg, about 25 mcg, about 37.5 mcg, about 50 mcg, or about 62.5 mcg of mometasone or its salt (such as about 50 mcg mometasone furoate) and olopatadine hydrochloride equivalent to about 300 mcg, about 450 mcg, about 600 mcg, about 750 mcg, or about 900 mcg of olopatadine (such as about 665 mcg olopatadine hydrochloride)). In one embodiment, each spray provides about 665 mcg olopatadine hydrochloride and about 25 mcg mometasone furoate. The administration may provide relief from one or more symptoms within 30 minutes, such as within 15 minutes or 10 minutes. In another embodiment, the administration may provide relief from one or more symptoms of allergic rhinitis (such as nasal symptoms) in a subject exposed to an environmental exposure chamber (EEC) (such as one with ragweed pollen at a concentration of 3500±500 particles/m3 for 6 hours) in less than 15 minutes, such as within about 10 minutes.

The pediatric human subject may previously been treated with mometasone or its salt alone or olopatadine or its salt alone. In such human subjects, the methods described herein may include the step of discontinuing treatment with the monotherapy of mometasone or its salt or olopatadine or its salt prior to initiating nasal administration of the fixed dose pharmaceutical composition of mometasone or its salt and olopatadine or its salt.

In one embodiment, the methods herein provide faster onset of action for relief of nasal symptoms in the subject. In another embodiment, the methods herein provide faster onset of action for relief of ocular symptoms in the subject.

In one preferred embodiment, the method comprises nasally administering to a human subject twice daily, one spray per nostril of a fixed-dose pharmaceutical composition comprising 25 mcg mometasone furoate and 665 mcg olopatadine hydrochloride.

In one embodiment, the pharmaceutical composition provides faster onset of action for relief of nasal symptoms in the subject. In another embodiment, the pharmaceutical composition provides faster onset of action for relief of ocular symptoms in the subject.

Described is a method of treating a pediatric human subject suffering from allergic rhinitis comprising the step of administering to the subject a pharmaceutical composition for twice daily nasal administration of one spray per nostril, wherein (i) the pharmaceutical composition provides an onset of action within 15 minutes for the treatment of allergic rhinitis and (ii) each spray of the pharmaceutical composition comprises about 25 mcg of mometasone furoate and about 665 mcg of olopatadine hydrochloride.

Described is a method of treating a pediatric human subject suffering from allergic rhinitis comprising the steps of:
(a) prescribing to a pediatric human subject a fixed-dose pharmaceutical composition for twice daily nasal administration of one spray per nostril, wherein the fixed-dose pharmaceutical composition comprises mometasone or its salt (such as mometasone furoate) and olopatadine or its salt (such as olopatadine hydrochloride), and each spray comprises mometasone or its salt and olopatadine or its salt in a weight ratio of about 1:5 to about 1:60 (such as a weight ratio of about 1:12 to about 1:53, about 1:13.3 to about 1:50, or from about 1:18 to about 1:40) (based on the equivalent weight of olopatadine free base) (for example, each spray comprises about 12.5 mcg, about 25 mcg, about 37.5 mcg, about 50 mcg, or about 62.5 mcg of mometasone or its salt (such as about 50 mcg mometasone furoate) and olopatadine hydrochloride equivalent to about 300 mcg, about 450 mcg, about 600 mcg, about 750 mcg, or about 900 mcg of olopatadine (such as about 665 mcg olopatadine hydrochloride)) (preferably each spray comprises about 665 mcg of olopatadine hydrochloride and about 25 mcg mometasone furoate),
   the prescribing being performed in response to (i) marketing of the pharmaceutical composition as (A) providing faster onset of action (such as an onset of action within less than 30 minutes, such as within 15 minutes, such as within 10 minutes) for relief of one or more symptoms (e.g., nasal symptoms) of allergic rhinitis than nasal administration of mometasone or its salt (such as 25 mcg mometasone furoate) or olopatadine or its salt (such as 665 mcg olopatadine hydrochloride) alone, (B) providing relief of one or more symptoms of allergic rhinitis within 15 minutes (or 30 minutes), and (ii) diagnosis of the human subject as suffering from allergic rhinitis, or (C) providing faster onset of action (such as within 15 minutes, such as within about 10 minutes) for relief of one or more symptoms (e.g., nasal symptoms) of allergic rhinitis in subjects exposed to an environmental exposure chamber (EEC) (such as one with ragweed pollen at a concentration of 3500±500 particles/m³ for 6 hours) than nasal administration of the mometasone or its salt or the olopatadine or its salt alone. (D) providing relief from one or more symptoms of allergic rhinitis (such as nasal symptoms) in subjects exposed to an environmental exposure chamber (EEC) (such as one with ragweed pollen at a concentration of 3500±500 particles/m³ for 6 hours) within 15 minutes, such as within about 10 minutes; and
(b) administering the prescribed pharmaceutical composition to the subject. In one preferred embodiment, each spray of the fixed-dose pharmaceutical composition provides 25 mcg mometasone furoate and 665 mcg olopatadine hydrochloride.

Described is a method of treating a pediatric human subject suffering from allergic rhinitis (such as seasonal allergic rhinitis or perennial allergic rhinitis) comprising the step of administering to the subject a prescribed fixed-dose pharmaceutical composition for twice daily nasal administration of one spray per nostril, where the fixed-dose pharmaceutical composition comprises mometasone or its salt (such as mometasone furoate) and olopatadine or its salt (such as olopatadine hydrochloride), and each spray comprises mometasone or its salt and olopatadine or its salt in a weight ratio of about 1:5 to about 1:60 (such as a weight ratio of about 1:12 to about 1:53, about 1:13.3 to about 1:50, or from about 1:18 to about 1:40) (based on the equivalent weight of olopatadine free base) (for example, each spray comprises about 12.5 mcg, about 25 mcg, about 37.5 mcg, about 50 mcg, or about 62.5 mcg of mometasone or its salt (such as about 50 mcg mometasone furoate) and olopatadine hydrochloride equivalent to about 300 mcg, about 450 mcg, about 600 mcg, about 750 mcg, or about 900 mcg of olopatadine (such as about 665 mcg olopatadine hydrochloride)). In one embodiment, each spray provides about 665 mcg olopatadine hydrochloride and about 25 mcg mometasone furoate. The pharmaceutical composition is prescribed in response to (a) marketing of the pharmaceutical composition as (A) providing faster onset of action (such as an onset of action within less than 30 minutes, such as within 15 minutes, such as within 10 minutes) for relief of symptoms (e.g., nasal symptoms) of allergic rhinitis than nasal administration of mometasone or its salt (such as 50 mcg mometasone furoate) or olopatadine or its salt (such as 665 mcg olopatadine hydrochloride) alone or (B) providing relief of one or more symptoms of allergic rhinitis within 15 minutes (or 30 minutes), and (b) a diagnosis of the subject as suffering from allergic rhinitis. In one preferred embodiment, each spray of the fixed-dose pharmaceutical composition provides 25 mcg mometasone furoate and 665 mcg olopatadine hydrochloride.

In one embodiment of any of the methods described herein, the allergic rhinitis is selected from perennial allergic rhinitis, persistent allergic rhinitis, seasonal allergic rhinitis, and nasal and/or non-nasal symptoms associated therewith. In one preferred embodiment, of any of the methods described herein, the allergic rhinitis is seasonal allergic rhinitis. In another preferred embodiment, of any of the methods described herein, the allergic rhinitis is perennial allergic rhinitis.

In one embodiment, the subject suffers from persistent allergic rhinitis and is treated for 4 or 6 weeks.

In another embodiment, the subject exhibits a positive skin prick test to an allergen. Alternately, the subject may also exhibit positive blood tests showing an allergy.

In yet another embodiment, the method involves no significant treatment-related adverse effects in the subject after 1 or 2 weeks treatment.

Described is a method of treating seasonal allergic rhinitis and/or nasal symptoms associated with seasonal allergic rhinitis in a pediatric subject in need thereof comprising nasally administering to the subject a combination (e.g., synergistic combination) comprising mometasone furoate and olopatadine hydrochloride, wherein the combination is in the form of a pharmaceutical composition comprising mometasone furoate and olopatadine hydrochloride in a weight ratio of about 1:5 to about 1:60 or from about 1:13.3 to about 1:53.2 (based on the equivalent weight of olopatadine free base). Described is a method of treating allergic rhinitis in a human in need thereof comprising nasally administering to the human an effective amount of a fixed-dose pharmaceutical composition comprising mometasone or its salt and olopatadine or its salt, wherein (i) the composition is nasally admnistered as 1 sprays per nostril of the human twice daily, and (ii) each spray comprises mometasone or its salt and olopatadine or its salt in a weight ratio of about 1:5 to about 1:60 or from about 1:13.3 to about 1:53.2 (based on the equivalent weight of olopatadine free base). In one embodiment, each spray provides about 665 mcg olopatadine hydrochloride and about 25 mcg mometasone furoate. The method may provide reduction in at least one treatment-related adverse effect (e.g. epistaxis and somnolence) relative to the use of the mometasone or olopatadine alone. For example, the method may provide effective treatment of seasonal allergic rhinitis and/or nasal symptoms associated with seasonal allergic rhinitis in a subject with a reduced incidence of drowsiness and nose bleeds. In another embodiment, the method may provide effective treatment of seasonal allergic rhinitis and/or nasal symptoms associated with seasonal allergic rhinitis in a subject without significant drowsiness and/or inducing nose bleeds.

Described is a method of treating perennial allergic rhinitis and/or nasal symptoms associated with perennial allergic rhinitis in a pediatric subject in need thereof comprising nasally administering to the subject a combination (e.g., synergistic combination) comprising mometasone furoate and olopatadine hydrochloride, wherein the combination is in the form of a pharmaceutical composition comprising mometasone furoate and olopatadine hydrochloride in a weight ratio of about 1:5 to about 1:60 or from about 1:13.3 to about 1:53.2 (based on the equivalent weight of olopatadine free base). In one embodiment, each spray provides about 665 mcg olopatadine hydrochloride and about 25 mcg mometasone furoate.

In one aspect of this embodiment, each spray comprises about 25 mcg mometasone furoate and olopatadine hydrochloride equivalent to about 600 mcg olopatadine. In one aspect of this embodiment, the pharmaceutical composition is a suspension comprising mometasone or its salt in particulate form and olopatadine or its salt in solution. In one aspect of this embodiment, the composition is administered for a period of at least 1 week as 1 spray per nostril twice daily. In yet another aspect of this embodiment, the allergic rhinitis is selected from perennial allergic rhinitis, persistent allergic rhinitis, seasonal allergic rhinitis, and nasal and/or non-nasal symptoms associated therewith. In a preferred aspect, the allergic rhinitis is seasonal allergic rhinitis and/or nasal symptoms associated therewith. In yet another aspect of this embodiment, (i) the total nasal symptoms score (TNSS) of the human is reduced by at least 50 % from baseline after 2 weeks treatment, and/or (ii) total ocular symptom score (TOSS) of the human is reduced by at least 40 % from baseline after 2 weeks treatment, and/or (iii) no significant treatment-related adverse effects are observed in the human after 2 weeks treatment. In one aspect of the said embodiment, the human exhibits a positive skin prick test to an allergen. In one embodiment, the treatment-related adverse effects include somnolence or epistaxis or a combination thereof.

The fixed-dose pharmaceutical composition comprising mometasone or its salt and olopatadine or its salt may be nasally administered to the pediatric human subject as 1 spray per nostril twice daily for a period of at least 1 week or at least 2 weeks.

In another embodiment of any of the methods described herein, the subject is a pediatric subject (e.g., ≥ 2 to < 12 years of age, such as ≥ 6 to < 12 years of age or ≥ 6 months to < 6 years of age) and the fixed-dose pharmaceutical composition is nasally administered 1 spray per nostril twice daily. In one embodiment, each spray of the fixed-dose pharmaceutical composition provides 665 mcg olopatadine hydrochloride and 25 mcg mometasone furoate (e.g., with the formulation of Example 9). In yet another embodiment, the composition is nasally administered for a period of at least 1 week, such as for a period of at least 2 weeks, for a period of at least 4 weeks, for a period of at least 8 weeks or for a period of at least 12 weeks. In one embodiment, the subject is 2 to 11 years of age. In another embodiment, the subject is 6 to 11 years of age. In yet another embodiment, the subject is 2 to 5 years of age.

Yet another embodiment is a method of treating symptoms associated with allergic rhinitis in a pediatric human subject (e.g., ≥ 2 to < 12 years of age, such as ≥ 6 to <12 years of age or ≥ 6 months to < 6 years of age) in need thereof comprising twice daily nasal administration of a single dose of a pharmaceutical composition comprising mometasone furoate and olopatadine hydrochloride, where a single dose of the composition provides about 1330 mcg olopatadine hydrochloride and about 50 mcg mometasone furoate. In one preferred embodiment, a single dose comprises one spray per nostril of a pharmaceutical composition, where each spray provides 665 mcg olopatadine hydrochloride and 25 mcg mometasone furoate (e.g., with the formulation of Example 9). The allergic rhinitis can be, for example, seasonal allergic rhinitis or perennial allergic rhinitis. In one embodiment, the subject is 2 to 11 years of age. In another embodiment, the subject is 6 to 11 years of age. In yet another embodiment, the subject is 2 to 5 years of age.

Yet another embodiment is a method of treating symptoms associated with allergic rhinitis in a pediatric human subject (e.g., ≥ 2 to < 12 years of age, such as ≥ 6 to <12 years of age or ≥ 6 months to < 6 years of age) in need thereof comprising nasally administering twice daily, one spray per nostril of a fixed-dose pharmaceutical composition comprising mometasone furoate and olopatadine hydrochloride, where each spray provides 665 mcg olopatadine hydrochloride and 25 mcg mometasone furoate (e.g., with the formulation of Example 9). The allergic rhinitis can be, for example, seasonal allergic rhinitis or perennial allergic rhinitis. In one embodiment, the subject is 2 to 11 years of age. In another embodiment, the subject is 6 to 11 years of age. In yet another embodiment, the subject is 2 to 5 years of age.

Described is a method of treating seasonal allergic rhinitis and/or nasal symptoms associated with seasonal allergic rhinitis in a pediatric human in need thereof comprising nasally administering to the human a combination (e.g., synergistic combination) comprising mometasone furoate and olopatadine hydrochloride, wherein the combination is in the form of a pharmaceutical composition comprising mometasone furoate in particulate form and olopatadine hydrochloride in solution in a weight ratio of about 1:13.3 to about 1:53.2 (based on the equivalent weight of olopatadine free base), and wherein the composition is administered as 1 spray per nostril of the human, twice daily for a period of at least 1 week wherein the method provides reduction of at least one treatment-related adverse effect.

In one aspect of this embodiment, the composition is administered twice daily for a period of 2 weeks. In another aspect of this embodiment, each spray comprises about 25 mcg of mometasone furoate and about 665 mcg of olopatadine hydrochloride. In yet another aspect of this embodiment, (i) the total nasal symptoms score (TNSS) of the human is reduced by at least 50 % from baseline after 2 weeks treatment, and/or (ii) total ocular symptom score (TOSS) of the human is reduced by at least 40 % from baseline after 2 weeks treatment, and/or (iii) no significant treatment-related adverse effects are observed in the human after 2 weeks treatment. In one aspect of the said embodiment, the human exhibits a positive skin prick test to an allergen. Preferably, the treatment-related adverse effects include somnolence or epistaxis or a combination thereof.

In an aspect of the invention, the fixed-dose pharmaceutical composition may be administered for a period of about 1 week, 2 weeks, 4 weeks, 6 weeks or 8 weeks. Preferably, the fixed-dose pharmaceutical composition, is administered as 1 spray per nostril of the pediatric human subject, twice daily for a period of 1 week or 2 weeks. In another aspect of the embodiment, each spray of the composition comprises olopatadine hydrochloride equivalent to about 600 mcg of olopatadine and about 25 mcg of mometasone furoate. Preferably, each spray of the composition comprises about 665 mcg of olopatadine hydrochloride (equivalent to about 600 mcg of olopatadine) and about 25 mcg of mometasone furoate. In yet another aspect, the composition does not have unpleasant odor and taste. In yet another aspect of the embodiment, the total nasal symptoms score (TNSS) of the human subject is reduced by at least 40 % or at least 50 % from baseline after 1 or 2 weeks treatment. In yet another aspect of the embodiment, the total ocular symptom score (TOSS) of the human is reduced by at least 30 % or at least 40 % from baseline after 1 or 2 weeks treatment. In yet another aspect of the embodiment, the said method provides reduction in one or more treatment-related adverse effects. Preferably, the treatment-related adverse effects include somnolence or epistaxis or a combination thereof. In yet another aspect of the embodiment, the human subject is a patient exhibiting a positive skin prick test to an allergen.

Described is the use of mometasone or its salt and olopatadine or its salt in a weight ratio of about 1:5 to about 1:60 or from about 1:12 to about 1:53 for the manufacture of a fixed-dose pharmaceutical composition of the invention for the treatment of allergic rhinitis in a pediatric human subject in need thereof. In an aspect, the fixed-dose pharmaceutical composition is a suspension wherein mometasone or its salt is present in particle form (e.g., having a mean particle size of from about 1 to about 20 µm, or from about 1 to about 15 µm) and olopatadine or its salt is present in dissolved form. In yet another aspect the composition does not have unpleasant odor and taste.

Described is a method of reducing symptoms associated with rhinitis in a pediatric human subject in need thereof, the method comprising nasally administering to the subject an effective amount of a fixed dose pharmaceutical composition comprising mometasone or its salt and olopatadine or its salt in a weight ratio of about 1:5 to about 1:60 or from about 1:12 to about 1:53 for about 1 or 2 weeks.

Described is a method of reducing symptoms associated with allergic rhinitis in a pediatric human in need thereof comprising nasally administering to the human an effective amount of a fixed-dose pharmaceutical composition comprising mometasone or its salt and olopatadine or its salt, wherein (i) the composition is nasally administered as 1 spray per nostril of the human twice daily, and (ii) each spray comprises mometasone or its salt and olopatadine or its salt in a weight ratio of about 1:5 to about 1:60 or from about 1:12 to about 1:53 for about 1 or 2 weeks (based on the equivalent weight of olopatadine free base) and wherein the composition is administered as 1 spray per nostril of the human, twice daily for a period of at least 1 week, wherein the method provides reduction of at least one adverse event. In one aspect of this embodiment, each spray comprises about 25 mcg mometasone furoate and olopatadine hydrochloride equivalent to about 600 mcg olopatadine. In one aspect of this embodiment, the pharmaceutical composition is a suspension comprising mometasone or its salt in particulate form and olopatadine or its salt in solution. In one aspect of this embodiment, the composition is administered for a period of at least 1 week as 1 spray per nostril twice daily. In yet another aspect of this embodiment, the allergic rhinitis is selected from perennial allergic rhinitis, persistent allergic rhinitis, seasonal allergic rhinitis, and nasal and/or non-nasal symptoms associated therewith. In a preferred aspect, the allergic rhinitis is seasonal allergic rhinitis and/or nasal symptoms associated therewith. In yet another aspect of this embodiment, (i) the total nasal symptoms score (TNSS) of the human is reduced by at least 50 % from baseline after 2 weeks treatment, and/or (ii) total ocular symptom score (TOSS) of the human is reduced by at least 40 % from baseline after 2 weeks treatment, and/or (iii) no significant treatment-related adverse effects are observed in the human after 2 weeks treatment. In one aspect of the said embodiment, the human exhibits a positive skin prick test to an allergen. In one aspect of the said embodiment, the symptoms includes rhinorrhea, nasal congestion, nasal itching, sneezing, itching/burning eyes, tearing/watering eyes, redness of eyes, itching of ears or palate, coughing, ocular pruritus, excess lacrimation, headache, fatigue and malaise. In yet another aspect of the embodiment, the treatment-related adverse effects include somnolence or epistaxis or a combination thereof.

Described is a method of reducing eosinophil count in the nasal lavage of a subject by nasally administering an effective amount of a fixed-dose pharmaceutical composition comprising mometasone or its salt and olopatadine or its salt in a weight ratio of about 1:5 to about 1:60 (based on the equivalent weight of olopatadine free base), wherein the method provides a greater reduction in eosinophil count than that provided by mometasone or its salt or olopatadine or its salt when administered as a monotherapy. The eosinophil count can be measured by any known technique, such as with a hemocytometer.

Described is a method of reducing total cell count in the nasal lavage by nasally administering an effective amount of a fixed-dose pharmaceutical composition comprising mometasone or its salt and olopatadine or its salt in a weight ratio of about 1:5 to about 1:60 (based on the equivalent weight of olopatadine free base) wherein the method provides a greater reduction in total cell count than that provided by mometasone or its salt or olopatadine or its salt when administered as a monotherapy.

In another aspect, the present invention relates to a stable fixed dose, aqueous pharmaceutical composition for nasal administration to a pediatric human. The composition comprises mometasone or its salt and olopatadine or its salt. The pharmaceutical composition may be contained within a container suitable for nasal administration. In one embodiment, the pharmaceutical composition is contained within a spray bottle (such as a sprayer). The spray bottle may include a container for storing the pharmaceutical composition and a pump for spraying the pharmaceutical composition intranasally. In one embodiment, the container is capable of storing 30, 56, 60, or 120, or 240 doses. For example, a container storing 120 doses can provide a 1 month supply of the pharmaceutical composition (1 spray per nostril twice daily (4 sprays per day) for 30 days). A container storing 28 doses can provide a 1 week supply of the pharmaceutical composition (1 spray per nostril twice daily (4 sprays per day) for 7 days). In one embodiment, the container may be suitable for containing 4-5 mL (e.g., 4.5 mL or 28 doses), 9-10 mL of the pharmaceutical composition (e.g., 9 mL or 60 doses), or 18-19 mL (e.g., 18 mL or 120 doses) of the pharmaceutical composition.

One embodiment is a stable fixed dose, aqueous pharmaceutical composition (e.g., contained in a container) for nasal administration to a pediatric human, where the composition comprises about 0.001 % w/w to about 0.075 % w/w mometasone or its salt and about 0.5 % w/w to about 0.8 % w/w olopatadine or its salt. The pharmaceutical composition may be in the form of a solution or a suspension, but preferably the composition is in the form of a suspension (more preferably, a single phase suspension), wherein mometasone or its salt is present in particle form and olopatadine or its salt is present in dissolved form. In one aspect, the mometasone or its salt and olopatadine or its salt are present in a weight ratio of about 1:3 to about 1:106, or from about 1:5 to about 1:53, or preferably from about 1:5 to about 1:36.

The composition preferably also includes a hydrocolloid. In one embodiment, the composition is a suspension and includes a hydrocolloid in a sufficient amount to prevent or inhibit phase separation (i.e., separation of the particles and solution) after 3 or 6 months of storage at 25 ± 2°C and 60% ± 5 % relative humidity (RH) or at 40 ± 2°C and 75% ± 5 % RH. In one embodiment, the aqueous pharmaceutical composition is a single phase suspension which remains a single phase suspension even after 3 or 6 months of storage at 25 ± 2°C and 60% ± 5 % RH or at 40 ± 2°C and 75% ± 5 % RH.

Another embodiment is a stable fixed dose, aqueous pharmaceutical composition (e.g., contained in a container) for nasal administration to a pediatric human, where the composition comprises about 0.001 % w/w to about 0.075 % w/w mometasone furoate monohydrate and about 0.5 % w/w to about 0.8 % w/w olopatadine hydrochloride.

Yet another embodiment is a stable fixed dose, aqueous pharmaceutical suspension composition (e.g., contained in a container) for nasal administration to a human, where the composition comprises about 0.025 % w/w to about 0.05 % w/w mometasone or its salt, about 0.6 % w/w to about 0.7 % w/w olopatadine or its salt and a hydrocolloid.

Yet another embodiment is a stable fixed dose, aqueous pharmaceutical suspension composition (e.g., contained in a container) for nasal administration to a pediatric human, where the composition comprises about 0.025 % w/w to about 0.05 % w/w mometasone or its salt, about 0.6 % w/w to about 0.7 % w/w olopatadine or its salt and a hydrocolloid which includes carboxymethylcellulose sodium and xanthan gum. The hydrocolloid may be present at a concentration of at least about 0.1 % w/w of the composition.

One embodiment is a stable fixed dose, aqueous pharmaceutical suspension composition (e.g., contained in a container) for nasal administration to a pediatric human, comprising about 0.025 % w/w to about 0.05 % w/w mometasone furoate, about 0.6 % w/w to about 0.7 % w/w olopatadine hydrochloride and a hydrocolloid, where the hydrocolloid is xanthan gum. The xanthan gum may be present at a concentration of at least about 0.1 % w/w, or preferably between about 0.1 % w/w to about 3 % w/w of the composition.

Another embodiment is a stable fixed dose, aqueous pharmaceutical suspension composition (e.g., contained in a container) for nasal administration to a pediatric human, comprising about 0.025 % w/w to about 0.05 % w/w mometasone furoate, about 0.6 % w/w to about 0.7 % w/w olopatadine hydrochloride and a hydrocolloid, where the hydrocolloid comprises sodium carboxymethyl cellulose. The sodium carboxymethyl cellulose may be present at a concentration of at least about 0.1 % w/w, or preferably between about 0.1 % w/w to about 3 % w/w of the composition.

Yet another embodiment is a stable fixed dose aqueous pharmaceutical composition in the form of suspension (e.g., contained in a container) for nasal administration to a human, comprising mometasone or its pharmaceutically acceptable salt, olopatadine or its pharmaceutically acceptable salt, a hydrocolloid at a concentration of at least about 0.1 % w/w of the composition and a pharmaceutical acceptable excipient.

Suitable pharmaceutical acceptable excipients include, but are not limited to, chelating agents, preservatives, buffers, surfactants, isotonicity agents, taste masking agents, antioxidants, humectants, pH adjusting agents, and mixtures thereof.

In one embodiment, the pharmaceutical composition has a pH between about 3.3 and about 4.1, or between about 3.5 and about 3.9, or between about 3.5 to about 3.7. The inventors discovered that the olopatadine hydrochloride crystallizes out of the fixed dose combination aqueous suspension at a pH of 5 to 5.5. The olopatadine hydrochloride, however, remains dissolved in the aqueous suspension at a pH of about 3.3 to about 4.1.

The aqueous pharmaceutical composition preferably is substantially free of crystals of olopatadine hydrochloride. In one embodiment, the aqueous pharmaceutical composition contains less than 2%, less than 1%, less than 0.5%, less than 0.2%, or less than 0.1% of crystalline olopatadine hydrochloride, based on the 100% total weight of olopatadine hydrochloride in the composition. In another embodiment, the aqueous pharmaceutical composition is substantially free of crystals of olopatadine hydrochloride after 3 or 6 months of storage at 25 ± 2°C and 60% ± 5 % RH or at 40 ± 2°C and 75% ± 5 % RH. In yet another embodiment, the aqueous pharmaceutical composition contains less than 2%, less than1%, less than 0.5%, less than 0.2%, or less than 0.1% of crystalline olopatadine hydrochloride, based on the 100% total weight of olopatadine hydrochloride in the composition, after 3 or 6 months of storage at 25 ± 2°C and 60% ± 5 % RH or at 40 ± 2°C and 75% ± 5 % RH.

The osmolality of the pharmaceutical composition may range between about 200 mOsm/kg to about 400 mOsm/kg, or about 250 mOsm/kg to about 350 mOsm/kg.

The viscosity of the pharmaceutical composition may range from about 10 cps to about 200 cps or preferably from about 20 cps to about 150 cps. In one embodiment, the composition has a viscosity of from about 60 to about 150 cps, such as from about 90 to about 150 cps or from about 100 to about 150 cps. In another embodiment, the composition has a viscosity of from about 20 to about 60 cps.

In yet another aspect, the pharmaceutical composition is in the form of suspension and contains mometasone furoate in particles having a mean particle size in the range of from about 1 µm to about 20 µm, or preferably from about 1 µm to about 15 µm. In an aspect, the suspension pharmaceutical composition of the present invention has mean particle size of less than 15 µm when determined by microscopy technique.

In yet another aspect, the pharmaceutical composition, upon nasal administration (e.g., as a nasal spray) of a dose equivalent to 200 mcg of mometasone or its salt to a human, results in (a) an area under the curve (AUC)_{0-∞} for mometasone or its salt of about 50 pg•hr/mL to about 140 pg•hr/mL, preferably from about 68 pg•hr/mL to about 124 pg•hr/mL, (b) a Cₘₐₓ for mometasone or its salt of about 6.5 pg/mL to about 16 pg/ml, preferably from about 8.6 pg/mL to about 12.9 pg/ml, (c) a Tₘₐₓ for mometasone or its salt of about 15 minutes to about 120 minutes, or (d) any combination of any of the foregoing. In yet another aspect, the pharmaceutical composition, upon nasal administration (e.g., as a nasal spray) of a dose equivalent to 2400 mcg of olopatadine or its salt to a human, results in (a) an AUC_{0-∞} for olopatadine or its salt of about 42.5 ng•hr/mL to about 116.5 ng•hr/mL, preferably from about 56.7 ng•hr/mL to about 99.8 ng.hr/mL, (b) a Cₘₐₓ for olopatadine or its salt of about 10.3 ng/mL to about 24.1 ng/ml, preferably from about 13.8 ng/mL to about 20.7 ng/ml, (c) a Tₘₐₓ of about 15 minutes to about 120 minutes, or (d) any combination of any of the foregoing.

In yet another aspect, the pharmaceutical composition, upon nasal administration (e.g., as a nasal spray) of a dose equivalent to 100 mcg of mometasone or its salt (e.g., mometasone furoate) to a human for the first time, results in (a) an area under the curve (AUC)ₜₐᵤ for mometasone or its salt of about 12 pg•hr/mL to about 73 pg•hr/mL, preferably from about 20 pg•hr/mL to about 36 pg•hr/mL, (b) a Cₘₐₓ for mometasone or its salt of about 3 pg/mL to about 13 pg/ml, preferably from about 5 pg/mL to about 9 pg/ml, (c) a Tₘₐₓ for mometasone or its salt of about 15 minutes to about 120 minutes, or (d) any combination of any of the foregoing. In yet another aspect, the pharmaceutical composition, upon nasal administration (e.g., as a nasal spray) of a dose equivalent to 100 mcg of mometasone or its salt (e.g., mometasone furoate) to a human at steady state (e.g., after 8 days of twice daily administration of 100 mcg of mometasone or its salt), results in (a) an area under the curve (AUC)ₜₐᵤ for mometasone or its salt of about 26 pg•hr/mL to about 124 pg•hr/mL, preferably from about 40 pg•hr/mL to about 80 pg•hr/mL, (b) a Cₘₐₓ for mometasone or its salt of about 4 pg/mL to about 16 pg/ml, preferably from about 8 pg/mL to about 12 pg/ml, (c) a Tₘₐₓ for mometasone or its salt of about 15 minutes to about 120 minutes, or (d) any combination of any of the foregoing.

In yet another aspect, the pharmaceutical composition, when delivered as a nasal spray has spray characteristics comprising a spray pattern having a longest axis of about 15-75 mm, a shortest axis of about 10-65 mm, and an ellipticity of about 1-2.

Another embodiment is a stable fixed dose pharmaceutical composition in the form of a suspension (e.g., contained in a container) for nasal administration to a pediatric human, comprising mometasone furoate monohydrate, olopatadine hydrochloride and a hydrocolloid which comprises xanthan gum at a concentration of about 0.3 % w/w of the composition, wherein the composition has a pH between about 3.5 to about 3.9.

Yet another embodiment is a stable fixed dose pharmaceutical composition in the form of suspension (e.g., contained in a container) for nasal administration to a pediatric human, comprising mometasone furoate monohydrate, olopatadine hydrochloride and a hydrocolloid which comprises sodium carboxymethyl cellulose at a concentration of about 0.5 % w/w of the composition, wherein the composition has a pH between about 3.5 to about 3.9.

In a further embodiment, the stable fixed dose, aqueous pharmaceutical composition is contained in a sprayer, and on delivering a spray of the composition to a human nose results in a spray pattern having a longest axis of 15-75 mm, a shortest axis of 10-65 mm, and an ellipticity of 1-2.

One embodiment is a stable fixed dose, aqueous pharmaceutical composition comprising mometasone furoate monohydrate, olopatadine hydrochloride and optionally a hydrocolloid contained in a sprayer, wherein each spray of the aqueous pharmaceutical composition provides (i) mometasone furoate monohydrate equivalent to about 50 mcg of mometasone furoate and (ii) olopatadine hydrochloride equivalent to about 600 mcg olopatadine.

Yet another embodiment is a stable fixed dose, aqueous pharmaceutical composition comprising mometasone furoate monohydrate, olopatadine hydrochloride and optionally a hydrocolloid contained in a sprayer, wherein each spray of the aqueous pharmaceutical composition provides (i) mometasone furoate monohydrate equivalent to about 25 mcg of mometasone furoate and (ii) olopatadine hydrochloride equivalent to about 600 mcg olopatadine.

In an embodiment, the present invention relates to a stable fixed dose pharmaceutical aqueous suspension composition (e.g., contained in a container) for nasal administration to a pediatric human, where the composition comprises (1) about 0.025 % w/w mometasone furoate monohydrate, (2) about 0.665% w/w olopatadine hydrochloride, (3) a hydrocolloid selected from about 0.3 % w/w of xanthan gum and about 0.5 % w/w carboxymethyl cellulose sodium (4) about 0.02% w/w benzalkonium chloride, (5) about 0.4 % w/w sodium chloride, (6) about 0.01 % w/w di-sodium edetate, (7) about 0.94% w/w sodium phosphate heptahydrate, and (8) about 0.01 % w/w polysorbate 80.

Another embodiment is a stable fixed dose pharmaceutical aqueous suspension composition (e.g., contained in a container) for nasal administration to a pediatric human, where the composition comprises (1) about 0.050 % w/w mometasone furoate monohydrate, (2) about 0.665% w/w olopatadine hydrochloride, (3) a hydrocolloid selected from about 0.3 % w/w of xanthan gum and about 0.5 % w/w carboxymethyl cellulose sodium, (4) about 0.02% w/w benzalkonium chloride, (5) about 0.4 % w/w sodium chloride, (6) about 0.01 % w/w di-sodium edetate, (7) about 0.94% w/w sodium phosphate heptahydrate, and (8) about 0.01 % w/w polysorbate 80.

Yet another embodiment is a stable fixed dose pharmaceutical aqueous suspension composition (e.g., contained in a container) for nasal administration to a pediatric human, where the composition comprises (1) about 0.025 % w/w mometasone furoate monohydrate, (2) about 0.665% w/w olopatadine hydrochloride, (3) a hydrocolloid selected from about 0.3 % w/w of xanthan gum and about 0.5 % w/w carboxymethyl cellulose sodium, (4) about 1% w/w to about 1.2% w/w mixture of microcrystalline cellulose and carboxymethyl cellulose sodium, (5) about 0.02% w/w benzalkonium chloride, (6) about 0.4 % w/w sodium chloride, (7) about 0.01 % w/w disodium edetate, (8) about 0.94% w/w sodium phosphate heptahydrate, and (9) about 0.01 % w/w polysorbate 80.

Yet another embodiment is a stable fixed dose pharmaceutical aqueous suspension composition (e.g., contained in a container) for nasal administration to a pediatric human, where the composition comprises (1) about 0.050 % w/w mometasone furoate monohydrate, (2) about 0.665% w/w olopatadine hydrochloride, (3) a hydrocolloid selected from about 0.3 % w/w of xanthan gum and about 0.5 % w/w carboxymethyl cellulose sodium, (4) about 1% w/w to about 1.2% w/w mixture of microcrystalline cellulose and carboxymethyl cellulose sodium, (5) about 0.02% w/w benzalkonium chloride, (6) about 0.4 % w/w sodium chloride, (7) about 0.01 % w/w disodium edetate, (8) about 0.94% w/w sodium phosphate heptahydrate, and (9) about 0.01 % w/w polysorbate 80.

Yet another embodiment is a stable suspension suitable for nasal administration to a pediatric human, comprising (a) an aqueous solvent, (b) particles of mometasone furoate suspended in the solvent, the particles having a mean particle size of from about 1 to about 20 µm, (c) olopatadine hydrochloride dissolved in the solvent, and (d) a hydrocolloid, the suspension having a viscosity in the range of about 20 cps to about 150 cps. In one preferred embodiment, the suspension has a pH of about 3.5-3.9, and osmolality in the range of about 250 mOsm/kg to about 350 mOsm/kg. In one embodiment, the suspension further comprises a chelating agent, a preservative, a buffer, a surfactant, an isotonicity agent, and optionally a pH adjusting agent.

Preferably, the suspensions of the present invention have only one phase (i.e., they are preferably a single phase suspension).

In a further embodiment, the present invention relates to a stable fixed dose, aqueous pharmaceutical composition (e.g., contained in a container) for nasal administration comprising about 0.025 % w/w to about 0.05 % w/w mometasone or its salt and about 0.5 % w/w to about 0.8 % w/w olopatadine or its salt for the treatment of rhinitis in a human in need thereof.

Any of the aforementioned pharmaceutical compositions comprising mometasone or a salt thereof and olopatadine (or a salt thereof may be used in the aforementioned methods, such as method of treating allergic rhinitis.

Described is a kit comprising a stable fixed dose, aqueous pharmaceutical composition contained in a container, for nasal administration and a package insert containing instructions about the use of the pharmaceutical composition.

### DETAILED DESCRIPTION OF THE INVENTION

### Definitions

The terms used herein are defined as follows. If a definition set forth in the present application and a definition set forth earlier in a provisional application from which the present applications claims priority are in conflict, the definition in the present application shall control the meaning of the terms.

The "onset of action" is the point at which patients might reasonably expect to see a meaningful decrease in their allergic rhinitis symptoms (such as a meaningful decrease in reflective total nasal symptom score (rTNSS), instantaneous total nasal symptom score (iTNSS) or reflective total ocular symptom score (rTOSS)). Statistically, it is the first time point after initiation of treatment when the drug demonstrates a change greater than the placebo treatment from baseline in the primary efficacy endpoint. This statistically significant difference between drug and placebo is maintained for some period (e.g., for 4 hours) from this point onward. *See* "Guidance for Industry, Allergic Rhinitis: Clinical Development Programs for Drug Products", U.S. Department of Health and Human Services, Food and Drug Administration, Center for Drug Evaluation and Research (CDER), April 2000.

The term "faster onset of action" refers, in one embodiment, to a statistically significant faster reduction in one or more parameters associated with the treatment of allergic rhinitis in a subject, such as a statistically significant faster reduction in reflective total nasal symptom score (rTNSS), instantaneous total nasal symptom score (iTNSS) or reflective total ocular symptom score (rTOSS) of the subject.

The term "advertising" refers to notifying, informing, and/or apprising one or more individuals of information (e.g., the efficacy or time for onset of action of a pharmaceutical product for treating or reducing an indication), such as by mass media, including, but not limited to, newspaper, magazine, and internet advertisements, television commercials, and billboard signs. The term "advertising" as used herein also includes including a statement that the pharmaceutical product can treat or reduce the indication in the labeling for the pharmaceutical product.

The term "marketing" refers to the act or process of selling a product, including, but not limited to, any offer for sale or sale of a product, as well as advertising.

The term "effective amount" or "therapeutically effective amount" denotes an amount of an active ingredient that, when administered to a subject for treating allergic rhinitis, produces an intended therapeutic benefit in a subject. The term "active ingredient" (used interchangeably with "active" or "active substance" or "drug") as used herein includes mometasone or its salt and olopatadine or its salt.

By "salt" or "pharmaceutically acceptable salt", it is meant those salts and esters which are, within the scope of sound medical judgment, suitable for use in contact with the tissues of humans and lower animals without undue toxicity, irritation, and allergic response, commensurate with a reasonable benefit to risk ratio, and effective for their intended use. Representative acid additions salts include, but are not limited to, hydrochloride, hydrobromide, sulphate, bisulphate, acetate, oxalate, valerate, oleate, palmitate, stearate, laurate, borate, benzoate, lactate, phosphate, tosylate, mesylate, citrate, maleate, fumarate, furoate, succinate, tartrate, ascorbate, glucoheptonate, lactobionate, and lauryl sulphate salts. Representative alkali or alkaline earth metal salts include, but are not limited to, sodium, calcium, potassium and magnesium salts. Preferably, the mometasone salt is mometasone furoate (e.g., mometasone furoate monohydrate) and the olopatadine salt is olopatadine hydrochloride.

As used herein, the term "mometasone and its salt" also includes hydrates of mometasone and its salts, such as a monohydrate, for example mometasone furoate monohydrate.

All references to the weight of, or a weight ratio including, "olopatadine or its salt" or "olopatadine or its salts" are based on the equivalent weight of olopatadine free base, unless otherwise specified.

The term "treating" or "treatment" as used herein also covers the prophylaxis, mitigation, prevention, amelioration, or suppression of a disorder.

The term "synergistic" or "synergy" as used herein refers to a combination exhibiting an effect greater than would be expected from the sum of the effects of the individual components of the combination alone. The term "synergistic" or "synergy" with regard to the combination of mometasone or its salt with olopatadine or its salt which is used in the treatment allergic rhinitis (for example, in the form of a pharmaceutical composition, a combination product or a kit according to the invention) refers to an efficacy for the treatment of the allergic rhinitis that is greater than would be expected from the sum of their individuals effects. The advantages for the synergistic combinations of the present invention include, but are not limited to, exhibiting enhanced efficacy compared to each of the ingredients when used alone, lowering the required dose of one or more of the active ingredients of the combination, reducing the side effects of one or more of the active compounds of the combination and/or rendering one or more of the active ingredients more tolerable to the subject in need of treatment of the allergic rhinitis.

By "pharmaceutically acceptable excipients", it is meant any of the components of a pharmaceutical composition other than the actives and which are approved by regulatory authorities or are generally regarded as safe for human or animal use.

Unless indicated otherwise, a "subject", "pediatric subject", "pediatric human" or "pediatric human subject" refers to a human subject up to seventeen (17) years of age. In one embodiment, the subject is an infant (one month to two years of age). In another embodiment, the subject is a child (two years to twelve years of age). In yet another embodiment, the subject is an adolescent (twelve years to sixteen years of age). In yet another embodiment, the subject is two years to less than twelve years of age. In yet another embodiment, the subject is six years to less than twelve years of age. In yet another embodiment, the subject is up to sixteen years of age.

The term "allergic rhinitis" as used herein refers to an allergic and/or inflammatory disease of nasal mucosa, and includes, but is not limited to, inflammation and irritation of the mucous membrane inside the nose, and nasal and/or non-nasal symptoms associated therewith. Typically the allergic rhinitis includes persistent allergic rhinitis, perennial allergic rhinitis, seasonal allergic rhinitis, chronic rhinitis, rhinitis medicamentosa, vasomotor rhinitis, infective rhinitis, autonomic rhinitis, hormonal rhinitis, drug-induced rhinitis, atrophic rhinitis, and gustatory rhinitis. Preferably, the allergic rhinitis includes perennial allergic rhinitis, persistent allergic rhinitis, seasonal allergic rhinitis, and nasal and/or non-nasal symptoms associated therewith. More preferably, the allergic rhinitis includes seasonal allergic rhinitis, and nasal and/or non-nasal symptoms associated therewith.

In the context of present invention, the nasal and/or non-nasal symptoms associated with allergic rhinitis include, for example, sneezing, nasal itching, rhinorrhea (runny nose or excess nasal secretion), nasal congestion, coughing, ocular pruritis, excess lacrimation, headache, fatigue, and malaise.

### Methods of Treatment

Described is a use of a fixed dose combination of mometasone or its salt and olopatadine or its salt for the treatment of allergic rhinitis in a pediatric subject in need thereof. The inventors have surprisingly found that mometasone furoate and olopatadine hydrochloride act synergistically in the treatment of allergic rhinitis and their combination is more effective and provides better therapeutic value than treatment with either active ingredient alone.

Described is a method of treating allergic rhinitis in a pediatric subject in need thereof comprising nasally administering to the subject, an effective amount of a fixed-dose pharmaceutical composition comprising mometasone or its salt and olopatadine or its salt. Preferably, the composition is nasally administered as 1 spray per nostril of the subject twice daily. Each spray may comprise mometasone or its salt and olopatadine or its salt in a weight ratio of about 1:5 to about 1:60, from about 1:10 to about 1:55 or from about 1:12 to about 1:53 or from about 1:13.3 to about 1:50. Preferably, the weight ratio of mometasone or its salt to olopatadine or its salt ranges from about 1:18 to about 1:40 or from about 1:24 to about 1:26.6. In an embodiment, the fixed-dose pharmaceutical composition is a suspension wherein mometasone or its salt is present in particulate form and the olopatadine or its salt is present in dissolved form.

Described is a method of treating allergic rhinitis in a pediatric subject in need thereof comprising nasally administering to the subject an effective amount of a fixed-dose pharmaceutical composition comprising mometasone furoate monohydrate and olopatadine hydrochloride. The composition may be nasally administered as 1 spray per nostril of the subject twice daily. Each spray of the pharmaceutical composition may comprise olopatadine hydrochloride equivalent to about 300 mcg, about 450 mcg, about 600 mcg, about 750 mcg, or about 900 mcg of olopatadine, and about 12.5 mcg, about 25 mcg, about 37.5 mcg, about 50 mcg, or about 62.5 mcg of mometasone furoate. In one embodiment, each spray comprises olopatadine hydrochloride equivalent to about 600 mcg of olopatadine and about 25 mcg of mometasone furoate. In another embodiment, each spray comprises olopatadine hydrochloride equivalent to about 600 mcg of olopatadine and about 50 mcg of mometasone furoate. Preferably, each spray comprises about 665 mcg of olopatadine hydrochloride (equivalent to about 600 mcg of olopatadine) and about 25 mcg of mometasone furoate.

Allergic rhinitis in the context of present invention includes, but is not limited to, inflammation and irritation of the mucous membrane inside the nose, and nasal and/or non-nasal symptoms associated therewith. It includes, for example, persistent allergic rhinitis, perennial allergic rhinitis, seasonal allergic rhinitis, chronic rhinitis, rhinitis medicamentosa, vasomotor rhinitis, infective rhinitis, autonomic rhinitis, hormonal rhinitis, drug-induced rhinitis, atrophic rhinitis, and gustatory rhinitis. Preferably, the allergic rhinitis is selected from perennial allergic rhinitis, persistent allergic rhinitis, seasonal allergic rhinitis, and nasal and/or non-nasal symptoms associated therewith.

In the context of the present invention, symptoms associated with rhinitis includes rhinorrhea, nasal congestion, nasal itching, sneezing, itching/burning eyes, tearing/watering eyes, redness of eyes, itching of ears or palate, coughing, ocular pruritus, excess lacrimation, headache, fatigue and malaise.

Described is a method of treating allergic rhinitis in a pediatric human subject in need thereof comprising nasally administering to the human, a fixed-dose pharmaceutical composition comprising mometasone or its salt and olopatadine or its salt in a weight ratio of about 1:5 to about 1:60 or from about 1:12 to about 1:53 or from about 1:13.3 to about 1:50, or from about 1:18 to about 1:40, wherein the composition is nasally administered as 1 spray per nostril, twice daily. Each spray may comprise olopatadine hydrochloride equivalent to about 600 mcg of olopatadine and about 25 mcg of mometasone furoate. In an aspect of the embodiment, the composition is administered for about 1 week. In another aspect of the embodiment, the composition is administered for about 2 weeks.

In one embodiment, the total nasal symptoms score (TNSS) of the human subject is reduced by at least 40 %, preferably by at least 50 % from baseline after 1 or 2 weeks treatment. In another embodiment, the total ocular symptom score (TOSS) of the human subject is reduced by at least 30 %, preferably by at least 40 % from baseline after 1 or 2 weeks treatment. In an aspect of the invention, the total nasal symptoms score (TNSS) and the total ocular symptom score (TOSS) can be observed as instantaneous or reflective or both.

In the context of present invention, evaluation of total nasal symptoms scores (TNSS) include the sum of scores of nasal congestion, rhinorrhea, itching and sneezing from baseline to the end of treatment (e.g., 1 or 2 weeks). Further evaluation of the total ocular symptoms scores (TOSS) includes ocular itching, tearing/watery eyes and ocular redness from baseline to the end of treatment.

In one embodiment, the subject suffers from persistent allergic rhinitis and is treated for 4 or 6 weeks.

In another embodiment, the subject exhibits a positive skin prick test to an allergen. Alternately, the subject may also exhibit positive blood tests showing an allergy.

In yet another embodiment, the method involves no significant treatment-related adverse effects in the subject after 1 or 2 weeks treatment.

Described is a method of treating seasonal allergic rhinitis and/or nasal symptoms associated with seasonal allergic rhinitis in a pediatric subject in need thereof comprising nasally administering to the subject a synergistic combination comprising mometasone furoate and olopatadine hydrochloride, wherein the combination is in the form of a pharmaceutical composition comprising mometasone furoate and olopatadine hydrochloride in a weight ratio of about 1:5 to about 1:60 or from about 1:13.3 to about 1:53.2 (based on the equivalent weight of olopatadine free base).

Described is a method of treating allergic rhinitis in a pediatric human in need thereof comprising nasally administering to the human an effective amount of a fixed-dose pharmaceutical composition comprising mometasone or its salt and olopatadine or its salt, wherein (i) the composition is nasally administered as 1 spray per nostril of the human twice daily, and (ii) each spray comprises mometasone or its salt and olopatadine or its salt in a weight ratio of about 1:5 to about 1:60 or from about 1:13.3 to about 1:53.2 (based on the equivalent weight of olopatadine free base). In one embodiment, each spray provides about 665 mcg olopatadine hydrochloride and about 25 mcg mometasone furoate. The method may provide reduction of at least one treatment-related adverse effect (e.g. epistaxis and somnolence) relative to the use of the mometasone or olopatadine alone. For example, the method may provide effective treatment of seasonal allergic rhinitis and/or nasal symptoms associated with seasonal allergic rhinitis in a subject with a reduced incidence of drowsiness and nose bleeds. In another embodiment, the method may provide effective treatment of seasonal allergic rhinitis and/or nasal symptoms associated with seasonal allergic rhinitis in a subject without significant drowsiness and/or inducing nose bleeds.

Described is a method of treating perennial allergic rhinitis and/or nasal symptoms associated with perennial allergic rhinitis in a pediatric subject in need thereof comprising nasally administering to the subject a combination (e.g., a synergistic combination) comprising mometasone furoate and olopatadine hydrochloride, wherein the combination is in the form of a pharmaceutical composition comprising mometasone furoate and olopatadine hydrochloride in a weight ratio of about 1:5 to about 1:60 or from about 1:13.3 to about 1:53.2 (based on the equivalent weight of olopatadine free base). In one embodiment, each spray of the pharmaceutical composition provides about 665 mcg olopatadine hydrochloride and about 25 mcg mometasone furoate.

In one aspect of this embodiment, each spray comprises about 25 mcg mometasone furoate and olopatadine hydrochloride equivalent to about 600 mcg olopatadine. In one aspect of this embodiment, the pharmaceutical composition is a suspension comprising mometasone or its salt in particulate form and olopatadine or its salt in solution. In one aspect of this embodiment, the composition is administered for a period of at least 1 week as 1 spray per nostril twice daily. In yet another aspect of this embodiment, the allergic rhinitis is selected from perennial allergic rhinitis, persistent allergic rhinitis, seasonal allergic rhinitis, and nasal and/or non-nasal symptoms associated therewith. In a preferred aspect, the allergic rhinitis is seasonal allergic rhinitis and/or nasal symptoms associated therewith. In yet another aspect of this embodiment, (i) the total nasal symptoms score (TNSS) of the human is reduced by at least 50 % from baseline after 2 weeks treatment, and/or (ii) total ocular symptom score (TOSS) of the human is reduced by at least 40 % from baseline after 2 weeks treatment, and/or (iii) no significant treatment-related adverse effects are observed in the human after 2 weeks treatment. In one aspect of the said embodiment, the human exhibits a positive skin prick test to an allergen. In one aspect of the said embodiment, the treatment-related adverse effects include somnolence or epistaxis or a combination thereof.

Another embodiment is a method of treating allergic rhinitis in a pediatric subject, wherein the subject exhibits a positive skin prick test to an allergen. The skin prick test can be performed by pricking the skin with a needle or pin containing a small amount of ragweed allergen. In one embodiment, prior to administration of a combination of olopatadine and mometasone as described herein, the skin prick test was performed on the subject and resulted in a wheal diameter of at least 3 mm greater than a negative control such as saline.

The methods of treatment described herein can be administered to a pediatric subject without the subject exhibiting any significant treatment-related adverse effects, for example, after 1 or 2 weeks treatment.

The treatment related adverse effects in the context of the present invention may include, but are not limited to, eye disorders (e.g., conjunctivitis), gastrointestinal disorders (e.g., abdominal distension, diarrhoea, dyspepsia, dysphagia and gastric ulcer, haemorrhoidal haemorrhage, hyperchlorhydria, nausea and vomiting, and toothache), general disorders (e.g., fatigue, local swelling, peripheral oedema, pain and pyrexia), infections and infestations (e.g., oral herpes and upper respiratory tract infection), injury, poisoning and procedural complications, musculoskeletal and connective tissue disorders (e.g., arthralgia), nervous system disorders (e.g., disturbance in attention, dizziness, dysgeusia, somnolence and headache), reproductive system and breast disorder (e.g., dysmenorrhea) respiratory, thoracic and mediastinal disorders (e.g., epistaxis, dry throat, dyspnea, epistaxis, nasal congestion, nasal discomfort, respiratory tract haemorrhage, rhinorrhea, throat irritation, and upper-airway cough syndrome), skin and subcutaneous tissue disorders (e.g., rash and urticaria).

Described is a method of treating seasonal allergic rhinitis and/or nasal symptoms associated with seasonal allergic rhinitis in a pediatric subject in need thereof comprising nasally administering to the subject a synergistic combination comprising mometasone furoate and olopatadine hydrochloride, wherein the combination is in the form of a pharmaceutical composition comprising mometasone furoate and olopatadine hydrochloride in a weight ratio of about 1:5 to about 1:60 or from about 1:13.3 to about 1:53.2 (based on the equivalent weight of olopatadine free base). In one embodiment, each spray of the pharmaceutical composition provides about 665 mcg olopatadine hydrochloride and about 25 mcg mometasone furoate.
a) The fixed-dose pharmaceutical composition comprising mometasone or its salt and olopatadine or its salt may be nasally administered to the pediatric subject as 1 spray per nostril twice daily for a period of at least 1 week or 2 weeks.

Described is a method of treating seasonal allergic rhinitis and/or nasal symptoms associated with seasonal allergic rhinitis in a pediatric human in need thereof comprising nasally administering to the human a synergistic combination comprising mometasone furoate and olopatadine hydrochloride, wherein the combination is in the form of a pharmaceutical composition comprising mometasone furoate in particulate form and olopatadine hydrochloride in solution in a weight ratio of about 1:13.3 to about 1:53.2 (based on the equivalent weight of olopatadine free base), and wherein the composition is administered as 1 spray per nostril of the human, twice daily for a period of at least 1 week wherein the method provides reduction of at least one adverse event. In one aspect of this embodiment, the composition is administered twice daily for a period of 2 weeks. In another aspect of this embodiment, each spray comprises about 25 mcg of mometasone furoate and about 665 mcg of olopatadine hydrochloride. In yet another aspect of this embodiment, (i) the total nasal symptoms score (TNSS) of the human is reduced by at least 50 % from baseline after 2 weeks treatment, and/or (ii) total ocular symptom score (TOSS) of the human is reduced by at least 40 % from baseline after 2 weeks treatment, and/or (iii) no significant treatment-related adverse effects are observed in the human after 2 weeks treatment. In one aspect of the said embodiment, the human exhibits a positive skin prick test to an allergen. Preferably, the treatment-related adverse effects include somnolence or epistaxis or a combination thereof.

In an aspect of the invention, the fixed-dose pharmaceutical composition may be administered for a period of about 1 week, 2 weeks, 4 weeks, 6 weeks or 8 weeks. Preferably, the fixed-dose pharmaceutical composition, is administered as 1 spray per nostril of the pediatric subject, twice daily for a period of 1 week or 2 weeks. In another aspect of the embodiment, each spray of the composition comprises olopatadine hydrochloride equivalent to about 600 mcg of olopatadine and about 25 mcg of mometasone furoate. Preferably, each spray of the composition comprises about 665 mcg of olopatadine hydrochloride (equivalent to about 600 mcg of olopatadine) and about 25 mcg of mometasone furoate. In yet another aspect, the composition does not have unpleasant odor and taste. In yet another aspect of the embodiment, the total nasal symptoms score (TNSS) of the human subject is reduced by at least 40 % or at least 50 % from baseline after 1 or 2 weeks treatment. In yet another aspect of the embodiment, the total ocular symptom score (TOSS) of the human is reduced by at least 30 % or at least 40 % from baseline after 1 or 2 weeks treatment. In yet another aspect of the embodiment, the said method provides reduction in treatment-related adverse effects. Preferably, the treatment-related adverse effects include somnolence or epistaxis or a combination thereof. In yet another aspect of the embodiment, the human subject is a patient exhibiting a positive skin prick test to an allergen.

Described is the use of mometasone or its salt and olopatadine or its salt in a weight ratio of about 1:5 to about 1:60 or from about 1:12 to about 1:53 for the manufacture of a fixed-dose pharmaceutical composition of the invention for the treatment of allergic rhinitis in a pediatric subject in need thereof. In an aspect, the fixed-dose pharmaceutical composition is a suspension wherein mometasone or its salt is present in particle form (e.g., having a mean particle size of from about 1 to about 20 µm, or from about 1 to about 15 µm) and olopatadine or its salt is present in dissolved form. In yet another aspect, the composition does not have unpleasant odor and taste. In one embodiment, each spray of the pharmaceutical composition provides about 665 mcg olopatadine hydrochloride and about 25 mcg mometasone furoate.

Described is a method of reducing symptoms associated with rhinitis in a pediatric subject in need thereof, the method comprising nasally administering to the subject an effective amount of a fixed dose pharmaceutical composition comprising mometasone or its salt and olopatadine or its salt in a weight ratio of about 1:5 to about 1:60 or from about 1:12 to about 1:53 for about 1 or 2 weeks.

Described is a method of reducing symptoms associated with allergic rhinitis in a pediatric human in need thereof comprising nasally administering to the human an effective amount of a fixed-dose pharmaceutical composition comprising mometasone or its salt and olopatadine or its salt, wherein (i) the composition is nasally administered as 1 spray per nostril of the human twice daily, and (ii) each spray comprises mometasone or its salt and olopatadine or its salt in a weight ratio of about 1:5 to about 1:60 or from about 1:12 to about 1:53 for about 1 or 2 weeks. (based on the equivalent weight of olopatadine free base) and wherein the composition is administered as 1 spray per nostril of the human, twice daily for a period of at least 1 week, wherein the method provides reduction of at least one adverse event. In one aspect of this embodiment, each spray comprises about 25 mcg mometasone furoate and olopatadine hydrochloride equivalent to about 600 mcg olopatadine. In one aspect of this embodiment, the pharmaceutical composition is a suspension comprising mometasone or its salt in particulate form and olopatadine or its salt in solution. In one aspect of this embodiment, the composition is administered for a period of at least 1 week as 1 spray per nostril twice daily. In yet another aspect of this embodiment, the allergic rhinitis is selected from perennial allergic rhinitis, persistent allergic rhinitis, seasonal allergic rhinitis, and nasal and/or non-nasal symptoms associated therewith. In a preferred aspect, the allergic rhinitis is seasonal allergic rhinitis and/or nasal symptoms associated therewith. In yet another aspect of this embodiment, (i) the total nasal symptoms score (TNSS) of the human is reduced by at least 50 % from baseline after 2 weeks treatment, and/or (ii) total ocular symptom score (TOSS) of the human is reduced by at least 40 % from baseline after 2 weeks treatment, and/or (iii) no significant treatment-related adverse effects are observed in the human after 2 weeks treatment. In one aspect of the said embodiment, the human exhibits a positive skin prick test to an allergen. In one aspect of the said embodiment, the symptoms include rhinorrhea, nasal congestion, nasal itching, sneezing, itching/burning eyes, tearing/watering eyes, redness of eyes, itching of ears or palate, coughing, ocular pruritus, excess lacrimation, headache, fatigue and malaise. In yet another aspect of the embodiment, the treatment-related adverse effects include somnolence or epistaxis or a combination thereof.

The administration of the pharmaceutical composition may provide relief from one or more symptoms of allergic rhinitis (such as nasal symptoms or ocular symptoms) in a pediatric subject faster (e.g., an onset of action in less than 30 minutes, such as within about 15 minutes, such as within 10 minutes) than nasal administration of the mometasone or its salt or the olopatadine or its salt alone. In another embodiment, the administration may provide relief from one or more symptoms of allergic rhinitis (such as nasal symptoms) in a subject exposed to an environmental exposure chamber (EEC) (such as one with ragweed pollen at a concentration of 3500±500 particles/m³ for 6 hours) faster (e.g., an onset of action in less than 15 minutes, such as within about 10 minutes) than nasal administration of the mometasone or its salt or the olopatadine or its salt alone.

Described is a method for providing faster onset of relief of symptoms associated with allergic rhinitis in a pediatric human subject in need thereof comprising nasally administering twice daily, one sprays per nostril of a fixed-dose pharmaceutical composition comprising mometasone or its salt (e.g., mometasone furoate) and olopatadine its salt (e.g., olopatadine hydrochloride). This method may provide faster onset of relief of one or more symptoms compared to administration of the mometasone or its salt alone or olopatadine or its salt alone. Each spray may comprise mometasone or its salt and olopatadine or its salt in a weight ratio of about 1:5 to about 1:60 (such as a weight ratio of about 1:12 to about 1:53, about 1:13.3 to about 1:50, or from about 1:18 to about 1:40) (based on the equivalent weight of olopatadine free base) (for example, each spray comprises about 12.5 mcg, about 25 mcg, about 37.5 mcg, about 50 mcg, or about 62.5 mcg of mometasone or its salt (such as about 50 mcg mometasone furoate) and olopatadine hydrochloride equivalent to about 300 mcg, about 450 mcg, about 600 mcg, about 750 mcg, or about 900 mcg of olopatadine (such as about 665 mcg olopatadine hydrochloride)). In one embodiment, each spray of the pharmaceutical composition provides about 665 mcg olopatadine hydrochloride and about 25 mcg mometasone furoate. The administration may provide relief from one or more symptoms within 30 minutes, such as within 15 minutes or within 10 minutes. In another embodiment, the administration may provide relief from one or more symptoms of allergic rhinitis (such as nasal symptoms) in a subject exposed to an environmental exposure chamber (EEC) (such as one with ragweed pollen at a concentration of 3500±500 particles/m³ for 6 hours) in less than 15 minutes, such as within about 10 minutes.

Described is a method of treating a pediatric human subject suffering from allergic rhinitis comprising the step of administering to the subject a pharmaceutical composition for twice daily nasal administration of one spray per nostril, wherein (i) the pharmaceutical composition provides an onset of action within 15 minutes for the treatment of allergic rhinitis and (ii) each spray of the pharmaceutical composition comprises about 25 mcg of mometasone furoate and about 665 mcg of olopatadine hydrochloride.

Described is a method of treating a pediatric human subject suffering from allergic rhinitis comprising the steps of:
(a) prescribing to a pediatric subject a fixed-dose pharmaceutical composition for twice daily nasal administration of one spray per nostril, wherein the fixed-dose pharmaceutical composition comprises mometasone or its salt (such as mometasone furoate) and olopatadine or its salt (such as olopatadine hydrochloride), and each spray comprises mometasone or its salt and olopatadine or its salt in a weight ratio of about 1:5 to about 1:60 (such as a weight ratio of about 1:12 to about 1:53, about 1:13.3 to about 1:50, or from about 1:18 to about 1:40) (based on the equivalent weight of olopatadine free base) (for example, each spray comprises about 12.5 mcg, about 25 mcg, about 37.5 mcg, about 50 mcg, or about 62.5 mcg of mometasone or its salt (such as about 50 mcg mometasone furoate) and olopatadine hydrochloride equivalent to about 300 mcg, about 450 mcg, about 600 mcg, about 750 mcg, or about 900 mcg of olopatadine (such as about 665 mcg olopatadine hydrochloride)),
   the prescribing being performed in response to (i) marketing of the pharmaceutical composition as (A) providing faster onset of action (such as an onset of action within less than 30 minutes, such as within 15 minutes, such as within 10 minutes) for relief of one or more symptoms (e.g., nasal symptoms) of allergic rhinitis than nasal administration of mometasone or its salt (such as 25 mcg mometasone furoate) or olopatadine or its salt (such as 665 mcg olopatadine hydrochloride) alone, (B) providing relief of one or more symptoms of allergic rhinitis within 15 minutes (or 30 minutes), and (ii) diagnosis of the human subject as suffering from allergic rhinitis, or (C) providing faster onset of action (such as within 15 minutes, such as within about 10 minutes) for relief of one or more symptoms (e.g., nasal symptoms) of allergic rhinitis in subjects exposed to an environmental exposure chamber (EEC) (such as one with ragweed pollen at a concentration of 3500±500 particles/m³ for 6 hours) than nasal administration of the mometasone or its salt or the olopatadine or its salt alone. (D) providing relief from one or more symptoms of allergic rhinitis (such as nasal symptoms) in subjects exposed to an environmental exposure chamber (EEC) (such as one with ragweed pollen at a concentration of 3500±500 particles/m³ for 6 hours) within 15 minutes, such as within about 10 minutes; and
(b) administering the prescribed pharmaceutical composition to the subject. In one preferred embodiment, each spray of the fixed-dose pharmaceutical composition provides 25 mcg mometasone furoate and 665 mcg olopatadine hydrochloride.

Described is a method of treating a pediatric human subject suffering from allergic rhinitis (such as seasonal allergic rhinitis or perennial allergic rhinitis) comprising the step of administering to the subject a prescribed fixed-dose pharmaceutical composition for twice daily nasal administration of one spray per nostril, where the fixed-dose pharmaceutical composition comprises mometasone or its salt (such as mometasone furoate) and olopatadine or its salt (such as olopatadine hydrochloride), and each spray comprises mometasone or its salt and olopatadine or its salt in a weight ratio of about 1:5 to about 1:60 (such as a weight ratio of about 1:12 to about 1:53, about 1:13.3 to about 1:50, or from about 1:18 to about 1:40) (based on the equivalent weight of olopatadine free base) (for example, each spray comprises about 12.5 mcg, about 25 mcg, about 37.5 mcg, about 50 mcg, or about 62.5 mcg of mometasone or its salt (such as about 50 mcg mometasone furoate) and olopatadine hydrochloride equivalent to about 300 mcg, about 450 mcg, about 600 mcg, about 750 mcg, or about 900 mcg of olopatadine (such as about 665 mcg olopatadine hydrochloride)). The pharmaceutical composition is prescribed in response to (a) marketing of the pharmaceutical composition as (A) providing faster onset of action (such as an onset of action within less than 30 minutes, such as within 15 minutes, such as within 10 minutes) for relief of symptoms (e.g., nasal symptoms) of allergic rhinitis than nasal administration of mometasone or its salt (such as 50 mcg mometasone furoate) or olopatadine or its salt (such as 665 mcg olopatadine hydrochloride) alone or (B) providing relief of one or more symptoms of allergic rhinitis within 15 minutes (or 30 minutes), and (b) a diagnosis of the subject as suffering from allergic rhinitis. In one preferred embodiment, each spray of the fixed-dose pharmaceutical composition provides 25 mcg mometasone furoate and 665 mcg olopatadine hydrochloride.

Described is a method of reducing eosinophil count in the nasal lavage of a pediatric subject by nasally administering an effective amount of a fixed-dose pharmaceutical composition comprising mometasone or its salt and olopatadine or its salt in a weight ratio of about 1:5 to about 1:60 (based on the equivalent weight of olopatadine free base), wherein the method provides a greater reduction in eosinophil count than that provided by mometasone or its salt or olopatadine or its salt when administered as a monotherapy. The eosinophil count can be measured by any known technique, such as with a hemocytometer. In one embodiment, the method comprises nasally administering one spray per nostril, twice daily, of the pharmaceutical composition, where each spray comprises 25 mcg mometasone furoate and 665 mcg of olopatadine hydrochloride.

Yet another embodiment relates to a method of reducing total cell count in the nasal lavage of a pediatric subject by nasally administering an effective amount of a fixed-dose pharmaceutical composition comprising mometasone or its salt and olopatadine or its salt in a weight ratio of about 1:5 to about 1:60 (based on the equivalent weight of olopatadine free base) wherein the method provides a greater reduction in total cell count than that provided by mometasone or its salt or olopatadine or its salt when administered as a monotherapy. In one embodiment, the method comprises nasally administering one spray per nostril, twice daily, of the pharmaceutical composition, where each spray comprises 25 mcg mometasone furoate and 665 mcg of olopatadine hydrochloride.

In the context of the present invention, eosinophil count and total cell count can be measured using various instruments such as a hemocytometer. Sneezing response can be determined using instruments such as a whole body plethysmography (Buxco Research Systems, Wilmington, NC, USA).

In the context of the present invention, the fixed dose pharmaceutical composition comprising mometasone or its salt, olopatadine or its salt are preferably supplied in the form of nasal spray with one or more pharmaceutical acceptable excipients (e.g., chelating agents, preservatives, buffer, surfactants, isotonic agent, taste masking agents, suspending agents, humectants, antioxidants and diluents) in a container and a kit providing directions on its usage and administration. The pharmaceutical composition may, for example, have any one of the formulations described in International Patent Application No. PCT/IB2014/064251, filed September 4, 2014, U.S. Patent Application No. 14/483,837, filed September 11, 2014, U.S. Patent Application No. 14/662,128, filed March 18, 2015, U.S. Patent Application No. 15/183,534, filed June 15, 2016, and U.S. Patent Application No. 15/210,692, filed July 14, 2016.

The pharmaceutical composition can be nasally administered with a nasal spray device (e.g., one capable of delivery a mist spray in the nostrils of a subject for local action on nasal mucosa).

### Pharmaceutical Compositions

The pharmaceutical composition can contain an effective amount of mometasone or a salt thereof and olopatadine or a salt thereof. The effective amount of mometasone or its salt can range from about 0.01 mg to about 10 mg or preferably from about 0.02 mg to about 5 mg. The effective amount of olopatadine or its salt in the pharmaceutical composition can range from about 0.05 mg to about 20 mg, or preferably from about 0.1 mg to about 15 mg.

For daily administration by the nasal route, the effective amount of mometasone or its salt in the pharmaceutical composition can range from about 10 mcg to about 500 mcg, or preferably from about 20 mcg to about 400 mcg, and that for olopatadine or its salt can ranges from about 50 mcg to about 7000 mcg, or preferably from about 100 mcg to about 5400 mcg.

The term "average particle size" (or synonymously, "mean particle size") as used herein refers to the distribution of particles, wherein about 50 volume percent of all the particles measured have a size less than the defined average particle size value and about 50 volume percent of all particles measured have a particle size greater than the defined average particle size value. This can be identified by the term "D₅₀" or "d _{(0.5)}". The average particle size can be measured using various techniques such as microscopy, laser diffraction, photon correlation spectroscopy (PCS) and Coulter's principle.

The term "Cₘₐₓ" is the maximum concentration of a drug (e.g., mometasone or olopatadine) in the blood plasma.

The term "Tₘₐₓ" is the time at which the peak (maximum) blood plasma drug concentration is achieved.

The term "AUC_{0-∞}" is the mean area under the plasma concentration-time curve extrapolated to infinity. It is calculated as the arithmetic mean of the area under the plasma concentration-time curve from time 0 extrapolated to infinity.

The term AUCₜₐᵤ" is the area under the curve for a plasma, serum or blood concentration versus time curve of a drug reached by a given dose over one dosing interval at steady state. The area under the curve is measured for a time tau at steady state, where tau is the length of the dosing interval.

The term "hydrocolloid" refers to a colloid system wherein hydrophilic colloid particles (e.g., hydrophilic polymers) are dispersed in water. The hydrocolloid system can exist in gel state or sol (liquid) state. In suspension compositions, the hydrocolloids function as thickening, stabilizing and suspending agents. Non-limiting examples of hydrocolloids include xanthan gum, gum arabic, guar gum, locust bean gum, alginate, starch, agar-agar, carrageenan, gelatin, a mixture of microcrystalline cellulose (MCC) and sodium carboxymethyl cellulose (sodium CMC) (e.g., Avicel RC591^{®} (available from FMC Biopolymer, Philadelphia, PA), a mixture of MCC and sodium CMC with a sodium CMC content of 8.3-13.8%), and cellulose derivatives (e.g., carboxymethyl cellulose sodium). Preferably, the hydrocolloid includes xanthan gum or carboxymethylcellulose sodium.

Some embodiments of the present invention provide compositions comprising carboxymethylcellulose sodium. In some embodiments, the compositions comprise from about 0.08 to about 2% carboxymethylcellulose sodium. In some embodiments, the compositions comprise from about 0.1% w/w to about 1.5% w/w carboxymethylcellulose sodium. In some embodiments, the compositions comprise from about 0.12% w/w to about 1% w/w carboxymethylcellulose sodium. In some embodiments, the compositions comprise from about 0.15% w/w to about 0.75% w/w carboxymethylcellulose sodium. In some embodiments, the compositions comprise about 0.083% w/w carboxymethylcellulose sodium. In some embodiments, the compositions comprise 0.0830% w/w carboxymethylcellulose sodium. In some embodiments, the compositions comprise about 0.1% w/w carboxymethylcellulose sodium. In some embodiments, the compositions comprise 0.0996% w/w carboxymethylcellulose sodium. In some embodiments, the compositions comprise about 0.7% w/w carboxymethylcellulose sodium. In some embodiments, the compositions comprise 0.6656% w/w carboxymethylcellulose sodium.

The term "container" refers to single unit-dose container or multi-dose container. Suitable single unit-dose containers or multi-dose containers include, but are not limited to, glass, aluminum, polypropylene or high density polyethylene, for example, high density polyethylene containers produced using a blow-fill-seal manufacturing technique. In one embodiment, the container is a sprayer which delivers the pharmaceutical composition in the form of a fine mist. A sprayer generally includes a container containing a pharmaceutical composition, a pump sealed (e.g., hermetically engaged) with the container, an actuator removably receiving a top portion of the pump, and a cap removably engaged with the container and the actuator.

One embodiment is a stable fixed dose, aqueous pharmaceutical composition (e.g., contained in a container) for nasal administration to a human, where the composition comprises about 0.001 % w/w to about 0.075 % w/w mometasone or its salt and about 0.5 % w/w to about 0.8 % w/w olopatadine or its salt.

The pharmaceutical composition may be in the form of a solution or a suspension, but preferably the composition is in the form of a suspension (more preferably, a single phase suspension), wherein mometasone or its salt is present in particle form and olopatadine or its salt is present in dissolved form. The mometasone or its salt and olopatadine or its salt may be present at a weight ratio of about 1:3 to about 1:106, or from about 1:5 to about 1:53 or preferably from about 1:5 to about 1:36. In one embodiment, the weight ratio of mometasone or its salt and olopatadine or its salt in the composition is from about 1:10 to about 1:53 or from about 1:12 to about 1:30.

The composition preferably also includes a hydrocolloid. In one embodiment, the composition is a suspension and includes a hydrocolloid in a sufficient amount to prevent phase separation (i.e., separation of the particles and solution) after 3 or 6 months of storage at 25 ± 2°C and 60% ± 5 % relative humidity (RH) or at 40 ± 2°C and 75% ± 5 % RH. In one embodiment, the aqueous pharmaceutical composition is a single phase suspension which remains a single phase suspension even after 3 or 6 months of storage at 25 ± 2°C and 60% ± 5 % RH or at 40 ± 2°C and 75% ± 5 % RH.

Another embodiment is an aqueous pharmaceutical composition comprising (a) mometasone or its salt, (b) olopatadine or its salt, and (c) a fibrillar network comprising a cellulosic material. The fibrillar network may have interfibrillar spaces. In one embodiment, the interfibrillar spaces contain one or more mometasone particles. In some embodiments, the mometasone or its salt is present in particulate form having an average particle size of less than about 15 µm. In further embodiments, the mometasone particles are equidistantly, or substantially equidistantly, spaced within the fibrillar network. The average distance between adjacent mometasone particles may be spaced sufficiently to provide consistent delivery of a fixed amount (or an effective amount) of both the mometasone (or its salt) and olopatadine (or its salt), for example, for 30, 56, 60, 120, or 240 doses (e.g., by nasal spray administration). In some embodiments, the fibrillar network is at least partially responsible for the ability of the compositions of the present invention to provide consistent dosing of a fixed amount, or an effective amount, of the active ingredients to the target site. The olopatadine or its salt in such compositions may be in dissolved form.

The term 'stable' as used in connection with aqueous suspensions refers to a composition when shaken and then stored for at least 24 hours at ambient condition does not show phase separation on visual inspection. Preferably, such stable composition does not show phase separation for a period of at least 3 days, or at least 5 days, or at least 7 days. In one aspect, the 'stable' composition of the present invention shows, upon shaking (e.g., for 1 minute) and visual inspection, no lump formation and a total impurity content of no more than 1.0% after storage at ambient conditions (at about 25 °C and a relative humidity of about 60 %) for a period of at least 6 months.

In the context of the present invention, the drug content and impurities can be determined by various analytical techniques such as HPLC, LC-MS, TLC and the like.

It was observed that when various pharmaceutical compositions for nasal administration comprising mometasone or its salt and olopatadine or its salt were prepared, the compositions generally showed physical separation in the suspension composition. This physical instability further leads to lack of dose uniformity. Surprisingly, it was found that addition of a hydrocolloid at certain concentrations (e.g. at a concentration of at least about 0.1 % w/w) in the suspension composition yielded a physically stable composition (with no separation) suitable for nasal administration.

Another embodiment is a stable fixed dose, aqueous pharmaceutical suspension composition (e.g., contained in a container) for nasal administration to a human, where the composition comprises about 0.025 % w/w to about 0.05 % w/w mometasone or its salt, about 0.6 % w/w to about 0.7 % w/w olopatadine or its salt and a hydrocolloid.

Yet another embodiment is a stable fixed dose, aqueous pharmaceutical suspension composition (e.g., contained in a container) for nasal administration to a human, where the composition comprises about 0.025 % w/w to about 0.05 % w/w mometasone or its salt, about 0.6 % w/w to about 0.7 % w/w olopatadine or its salt and a hydrocolloid which includes carboxymethylcellulose sodium and xanthan gum. The hydrocolloid may be present at a concentration of at least about 0.1 % w/w of the composition.

Yet another embodiment is a stable fixed dose, aqueous pharmaceutical suspension composition (e.g., contained in a container) for nasal administration to a human, comprising about 0.025 % w/w to about 0.05 % w/w mometasone furoate, about 0.6 % w/w to about 0.7 % w/w olopatadine hydrochloride and a hydrocolloid which comprises xanthan gum. The xanthan gum may be present at a concentration of at least about 0.1 % w/w, or preferably between about 0.3 % w/w to about 3 % w/w of the composition.

Yet another embodiment is a stable fixed dose, aqueous pharmaceutical suspension composition (e.g., contained in a container) for nasal administration to a human, comprising about 0.025 % w/w to about 0.05 % w/w mometasone furoate, about 0.6 % w/w to about 0.7 % w/w olopatadine hydrochloride and a hydrocolloid which comprises sodium carboxymethyl cellulose. The sodium carboxymethyl cellulose may be present at a concentration of at least about 0.1 % w/w, or preferably between about 0.1 % w/w to about 3 % w/w of the composition.

Yet another embodiment is a stable fixed dose aqueous pharmaceutical composition in the form of suspension (e.g., contained in a container) for nasal administration to a human, comprising mometasone or its pharmaceutically acceptable salt, olopatadine or its pharmaceutically acceptable salt, a hydrocolloid (e.g., at a concentration of at least about 0.1 % w/w of the composition) and a pharmaceutical acceptable excipient.

It will also be appreciated to the skilled artisan that in order to improve the physical properties, appearances, or smells of the composition of the present invention, one or more further pharmaceutically acceptable excipients may be added as desired. Suitable pharmaceutical acceptable excipients include, but are not limited to, chelating agents, preservatives, buffers, surfactants, isotonicity agents, taste masking agents, antioxidants, humectants, pH adjusting agents, and any combination of any of the foregoing.

Suitable surfactants which can be used for preparing aqueous nasal spray composition may include one or more of anionic, cationic, non-ionic or zwitterionic surfactants.

Examples of suitable surfactants which can be employed in the aqueous nasal spray suspension may be selected from, but not limited to, polyethoxylated sorbitan derivatives such as polysorbates, their ether ethoxylates, produced by reaction of sorbitan esters with ethylene oxide, polyoxyethylene alkyl phenol, polyoxyethylene cetyl ether, polyoxyethylene alkyl-aryl ether, polyoxyethylene monolaurate, polyoxyethylene vegetable oil, polyoxyethylene sorbitan monolaurate, polyoxyethylene esters or mixed fatty and resin acids, polyoxyethylene sorbitol lanolin derivative, polyoxyethylene tridecylether, polyoxyethylene sorbitan esters of mixed fatty and resin acids, , polyoxyethylene sorbitan monostearate, polyoxyethylene sorbitan monooleate, polyoxyethylene monostearate, polyoxyethylene stearyl ether, polyoxyethylene oleyl ether, polyoxyethylene tridecyl ether, polyoxyethylene fatty alcohol, polyoxyethylene alkyl amine, polyoxyethylene glycol monopalmitate, polyoxyethylene sorbitan monopalmitate, polyoxyethylene cetyl ether, polyoxyethylene oxypropylene stearate, polyoxyethylene lauryl ether, polyoxyethylene lanolin derivative, sodium oleate, quaternary ammonium derivative, potassium oleate, N-cetyl N-ethyl morpholinium ethosulfate, sodium lauryl sulfate or mixtures thereof. Preferred surfactants are polyethoxylated sorbitan derivatives (such as polysorbate 80). The amount of surfactant may range from about 0.001% to about 1% w/w relative to the total weight of the composition.

In order to improve the ability of the aqueous nasal spray suspension to be tolerated on administration to the nasal mucous membrane, it is advantageous to formulate it as isotonic. The osmolality can be set by variation of the amounts of the substances present in the aqueous nasal spray suspension besides mometasone, olopatadine and any further substances present, and/or by addition of an isotonicity agent, preferably a physiologically tolerated salt, such as, for example, sodium chloride or potassium chloride, or a physiologically tolerated polyol, such as, for example, a sugar alcohol, in particular sorbitol or glycerol, in the concentration necessary for rendering isotonic.

Examples of suitable preservatives which can be employed in the aqueous nasal spray suspension include, but are not limited to, benzyl alcohol, quaternary ammonium halides, phenylcarbinol, thimerosal, and disodium edetate. Quaternary ammonium halide preservatives are preferred. Suitable quaternary ammonium halide preservatives include polyquaternium-1 and benzalkonium halides. Preferred benzalkonium halides include benzalkonium chloride and benzalkonium bromide. The amount of the preservative present in the aqueous nasal spray suspension may range from about 0.005 to about 0.2% w/w relative to the total weight of the composition. Preferably, the preservative is present at a concentration of about 0.02% w/w relative to the total weight of the composition.

Examples of suitable chelating agents which can be employed in the aqueous nasal spray suspension include, but are not limited to, edetate disodium (EDTA), edetate trisodium, edetate tetrasodium, and diethyleneamine pentaacetate, preferably EDTA. The amount of the chelating agent present in the aqueous nasal spray suspension of the present invention may range from about 0.0002 to about 0.5% w/w relative to the total weight of the composition.

Examples of suitable buffers which can be employed in the aqueous nasal spray suspension include, but are not limited to, citric acid, acetic acid, fumaric acid, hydrochloric acid, malic acid, nitric acid, phosphoric acid, propionic acid, sulfuric acid, tartaric acid, phosphate salts (e.g., dibasic sodium phosphate, such as dibasic sodium phosphate heptahydrate), or combinations thereof. The suspension of the present invention may comprise an amount of a buffer sufficient to maintain the pH of the composition to from about 3 to about 6. Preferably, the amount of buffer ranges from about 0.005% to about 1% w/w relative to the total weight of the composition.

Examples of suitable sweetener/taste masking agents which can be employed in the aqueous nasal spray suspension include, but are not limited to, sucralose, thaumatin (e.g., Talin^{(R)}), sucrose, saccharin (including salt forms such as sodium and calcium salts), fructose, glucose, dextrose, corn syrup, aspartame, acesulfame-K, xylitol, sorbitol, erythritol, ammonium glycyrrhizinate, neotame, mannitol, eucalyptus oil, camphor, and natural or artificial flavors or flavoring agents (for example menthol, mints, vanilla, orange, etc.), or combinations of two or more of such agents. A particularly preferred taste masking agent is sucralose. The amount of the sweetener/taste masking agent present in the aqueous nasal spray suspension may range from about 0.01 % to about 1% w/w relative to the total weight of the composition.

Examples of suitable antioxidants which can be employed in the aqueous nasal spray suspension include, but are not limited to, ascorbic acid, alpha-tocopherol (vitamin-E), butylated hydroxyanisole, butylated hydroxytoluene, glutathione, and any combination of any of the foregoing. The amount of the antioxidants present in the aqueous nasal spray composition may range from about 0.0002 % to about 0.5% w/w relative to the total weight of the composition.

Examples of suitable humectants which can be employed in the aqueous nasal spray suspension include, but are not limited to, glycerin, sorbitol, polyethylene glycol, propylene glycol or mixtures thereof, which are mixed with a suitable humectant vehicle such as water. The amount of humectant present in the aqueous nasal spray suspension may range from about 0.0002 % to about 0.5% w/w relative to the total weight of the composition.

Suitable pH adjusting agents include, but are not limited to, sodium hydroxide and hydrochloric acid.

In the context of present invention, the pharmaceutical stable fixed dose suspension composition for nasal administration may have a pH of between about 3.3 and about 4.1, or between about 3.5 and about 3.9. The inventors discovered that the olopatadine hydrochloride crystallizes out of the fixed dose combination aqueous suspension at a pH of 5 to 5.5. The olopatadine hydrochloride, however, remains dissolved in the aqueous suspension at a pH of about 3.3 to about 4.1.

The aqueous pharmaceutical composition preferably is substantially free of crystals of olopatadine hydrochloride. In one embodiment, the aqueous pharmaceutical composition contains less than 2%, less than 1%, less than 0.5%, less than 0.2%, or less than 0.1% of crystalline olopatadine hydrochloride, based on the 100% total weight of olopatadine hydrochloride in the composition. In another embodiment, the aqueous pharmaceutical composition is substantially free of crystals of olopatadine hydrochloride after 3 or 6 months of storage at 25 ± 2°C and 60% ± 5 % RH or at 40 ± 2°C and 75% ± 5 % RH. In yet another embodiment, the aqueous pharmaceutical composition contains less than 2%, less than 1%, less than 0.5%, less than 0.2%, or less than 0.1% of crystalline olopatadine hydrochloride, based on the 100% total weight of olopatadine hydrochloride in the composition, after 3 or 6 months of storage at 25 ± 2°C and 60% ± 5 % RH or at 40 ± 2°C and 75% ± 5 % RH.

The osmolality of the composition may range between about 200 mOsm/kg and about 400 mOsm/kg, or about 250 mOsm/kg and about 350 mOsm/kg. The viscosity of the composition may be about 10 cps to about 200 cps or preferably from about 20 cps to about 150 cps.

In yet another aspect, the pharmaceutical composition in the form of suspension and contains mometasone furoate in particles having mean particle size in the range of from about 1 µm to about 20 µm, or preferably from about 1 µm to about 15 µm. In an aspect, the suspension pharmaceutical composition of the present invention has mean particle size of less than 15 µm when determined by microscopy technique.

In yet another aspect, the pharmaceutical composition, upon nasal administration (e.g., as a nasal spray) of a dose equivalent to 200 mcg of mometasone or its salt to a human, results in (a) an area under the curve (AUC)_{0-∞} for mometasone or its salt of about 50 pg•hr/mL to about 140 pg•hr/mL, preferably from about 68 pg•hr/mL to about 124 pg•hr/mL, (b) a Cₘₐₓ for mometasone or its salt of about 6.5 pg/mL to about 16 pg/ml, preferably from about 8.6 pg/mL to about 12.9 pg/ml, (c) a Tₘₐₓ for mometasone or its salt of about 15 minutes to about 120 minutes, or (d) any combination of any of the foregoing.

In yet another aspect, the pharmaceutical composition, upon nasal administration (e.g., as a nasal spray) of a dose equivalent to 2400 mcg of olopatadine or its salt to a human, results in (a) an AUC_{0-∞} for olopatadine or its salt of about 42.5 ng•hr/mL to about 116.5 ng•hr/mL, preferably from about 56.7 ng•hr/mL to about 99.8 ng.hr/mL, (b) a Cₘₐₓ for olopatadine or its salt of about 10.3 ng/mL to about 24.1 ng/ml, preferably from about 13.8 ng/mL to about 20.7 ng/ml, (c) a Tₘₐₓ of about 15 minutes to about 120 minutes, or (d) any combination of any of the foregoing.

In yet another aspect, the pharmaceutical composition, upon nasal administration (e.g., as a nasal spray) of a dose equivalent to 100 mcg of mometasone or its salt (e.g., mometasone furoate) to a human for the first time, results in (a) an area under the curve (AUC)ₜₐᵤ for mometasone or its salt of about 12 pg•hr/mL to about 73 pg•hr/mL, preferably from about 20 pg•hr/mL to about 36 pg•hr/mL, (b) a Cₘₐₓ for mometasone or its salt of about 3 pg/mL to about 13 pg/ml, preferably from about 5 pg/mL to about 9 pg/ml, (c) a Tₘₐₓ for mometasone or its salt of about 15 minutes to about 120 minutes, or (d) any combination of any of the foregoing. In yet another aspect, the pharmaceutical composition, upon nasal administration (e.g., as a nasal spray) of a dose equivalent to 100 mcg of mometasone or its salt (e.g., mometasone furoate) to a human at steady state (e.g., after 8 days of twice daily administration of 100 mcg of mometasone or its salt), results in (a) an area under the curve (AUC)ₜₐᵤ for mometasone or its salt of about 26 pg•hr/mL to about 124 pg•hr/mL, preferably from about 40 pg•hr/mL to about 80 pg•hr/mL, (b) a Cₘₐₓ for mometasone or its salt of about 4 pg/mL to about 16 pg/ml, preferably from about 8 pg/mL to about 12 pg/ml, (c) a Tₘₐₓ for mometasone or its salt of about 15 minutes to about 120 minutes, or (d) any combination of any of the foregoing.

In yet another aspect, the pharmaceutical composition, when delivered as a nasal spray has a spray pattern having a longest axis of about 15-75 mm, a shortest axis of about 10-65 mm, and an ellipticity of about 1-2.

In the context of present invention, the viscosity can be determined by various known instruments such as a Dynamic stress rheometer or Brookfield viscometer. In a preferred embodiment, the viscosity is determined by a Brookfield viscometer by measuring torque transmission through a sample using a rotating spindle.

In another embodiment, the present invention relates to a stable fixed dose, aqueous pharmaceutical composition (e.g., contained in a container) for nasal administration to a human, where the composition comprises about 0.001 % w/w to about 0.075 % w/w mometasone furoate monohydrate and about 0.5 % w/w to about 0.8 % w/w olopatadine hydrochloride.

Another embodiment is a stable fixed dose pharmaceutical composition in the form of suspension (e.g., contained in a container) for nasal administration to a pediatric human subject, comprising mometasone furoate monohydrate, olopatadine hydrochloride and a hydrocolloid which comprises xanthan gum at a concentration of about 0.3 % w/w of the composition, wherein the composition has a pH between about 3.5 and about 3.9.

Yet another embodiment is a stable fixed dose pharmaceutical composition in the form of suspension (e.g., contained in a container) for nasal administration to a pediatric human subject, comprising mometasone furoate monohydrate, olopatadine hydrochloride and a hydrocolloid which comprises sodium carboxymethyl cellulose at a concentration of about 0.5 % w/w of the composition, wherein the composition has a pH between about 3.5 and about 3.9.

Yet another embodiment is a stable fixed dose pharmaceutical aqueous suspension composition (e.g., contained in a container) for nasal administration to a pediatric human subject, where the composition comprises (1) about 0.025 % w/w mometasone furoate monohydrate, (2) about 0.665% w/w olopatadine hydrochloride, (3) a hydrocolloid selected from about 0.3 % w/w of xanthan gum and about 0.5 % w/w carboxymethyl cellulose sodium, (4) about 0.02% w/w benzalkonium chloride, (5) about 0.4 % w/w sodium chloride, (6) about 0.01 % w/w di-sodium edetate, (7) about 0.94% w/w sodium phosphate heptahydrate, and (8) about 0.01 % w/w polysorbate 80.

Yet another embodiment is a stable fixed dose pharmaceutical aqueous suspension composition (e.g., contained in a container) for nasal administration to a pediatric human subject, where the composition comprises (1) about 0.050 % w/w mometasone furoate monohydrate, (2) about 0.665% w/w olopatadine hydrochloride, (3) a hydrocolloid selected from about 0.3 % w/w of xanthan gum and about 0.5 % w/w carboxymethyl cellulose sodium, (4) about 0.02% w/w benzalkonium chloride, (5) about 0.4 % w/w sodium chloride, (6) about 0.01 % w/w di-sodium edetate, (7) about 0.94% w/w sodium phosphate heptahydrate, and (8) about 0.01 % w/w polysorbate 80.

Yet another embodiment is a stable fixed dose pharmaceutical aqueous suspension composition (e.g., contained in a container) for nasal administration to a pediatric human subject, where the composition comprises (1) about 0.025 % w/w mometasone furoate monohydrate, (2) about 0.665% w/w olopatadine hydrochloride, (3) a hydrocolloid selected from about 0.3 % w/w of xanthan gum and about 0.5 % w/w carboxymethyl cellulose sodium, (4) about 1% w/w to about 1.2% w/w mixture of microcrystalline cellulose and carboxymethyl cellulose sodium, (5) about 0.02% w/w benzalkonium chloride, (6) about 0.4 % w/w sodium chloride, (7) about 0.01 % w/w di-sodium edetate, (8) about 0.94% w/w sodium phosphate heptahydrate, and (9) about 0.01 % w/w polysorbate 80.

Yet another embodiment is a stable fixed dose pharmaceutical aqueous suspension composition (e.g., contained in a container) for nasal administration to a pediatric human subject, where the composition comprises (1) about 0.050 % w/w mometasone furoate monohydrate, (2) about 0.665% w/w olopatadine hydrochloride, (3) a hydrocolloid selected from about 0.3 % w/w of xanthan gum and about 0.5 % w/w carboxymethyl cellulose sodium, (4) about 1% w/w to about 1.2% w/w mixture of microcrystalline cellulose and carboxymethyl cellulose sodium, (5) about 0.02% w/w benzalkonium chloride, (6) about 0.4 % w/w sodium chloride, (7) about 0.01 % w/w di-sodium edetate, (8) about 0.94% w/w sodium phosphate heptahydrate, and (9) about 0.01 % w/w polysorbate 80.

Yet another embodiment is a stable suspension suitable for nasal administration to a pediatric human subject, comprising (a) an aqueous solvent, (b) particles of mometasone furoate suspended in the solvent, the particles having a mean particle size of from about 1 to about 20 µm, (c) olopatadine hydrochloride dissolved in the solvent, and (d) a hydrocolloid, the suspension having a viscosity in the range of about 20 cps to about 150 cps. In one preferred embodiment, the suspension has a pH of about 3.5-3.9, and osmolality in the range of about 250 mOsm/kg to about 350 mOsm/kg. In one embodiment, the suspension further comprises a chelating agent, a preservative, a buffer, a surfactant, an isotonicity agent, and optionally a pH adjusting agent.

Preferably, the suspensions of the present invention have only one phase (i.e., they are preferably a single phase suspension).

Described is a to kit comprising a stable fixed dose, aqueous pharmaceutical composition of the present invention contained in a container for nasal administration and a package insert containing instructions about the use of said pharmaceutical composition. In one preferred embodiment, the container is part of a sprayer which has an actuator. When the actuator is actuated, the composition is delivered in the form of a spray.

In a further embodiment, the pharmaceutical composition is contained in a sprayer, and has, on deliver a spray of the composition to a human nose, a spray pattern having a longest axis of 15-75 mm, a shortest axis of 10-65 mm, and an ellipticity of 1-2.

In the context of present invention, the pharmaceutical composition when delivered as a nasal spray using a sprayer yields a specific spray pattern and spray droplet size. The spray pattern can be determined by various known techniques such as with an ADSA with NSPUA set up (Innova System) and the spray droplet size distribution can be determined by various known techniques such as with a Malvern Spraytec with NSPUA set up (Innova System).

The following describes a typical procedure for characterizing droplet size distribution of the spray - The sprayer is loaded with a composition as described above and primed by an actuating pump via an actuator until a fine mist appears out of the nozzle of the sprayer. A commercially available laser diffraction instrument is arranged so that the nozzle is about 3 cm or 6 cm below the laser beam of the laser diffraction instrument. The pump is actuated with a conventional mechanical actuator using a constant force. The resulting spray of the composition crosses the laser beam. Data are collected for D₁₀, D₅₀, D₉₀, SPAN, and % Volume <10 µm. The average values for each of these parameters for three sprays are calculated.

One embodiment is a stable fixed dose, aqueous pharmaceutical composition comprising mometasone furoate monohydrate, olopatadine hydrochloride and optionally a hydrocolloid contained in a sprayer, wherein each spray of the aqueous pharmaceutical composition provides (i) mometasone furoate monohydrate equivalent to about 50 mcg of mometasone furoate and (ii) olopatadine hydrochloride equivalent to about 600 mcg olopatadine.

Yet another embodiment is a stable fixed dose, aqueous pharmaceutical composition comprising mometasone furoate monohydrate, olopatadine hydrochloride and optionally a hydrocolloid contained in a sprayer, wherein each spray of the aqueous pharmaceutical composition provides (i) mometasone furoate monohydrate equivalent to about 25 mcg of mometasone furoate and (ii) olopatadine hydrochloride equivalent to about 600 mcg olopatadine.

The stable aqueous nasal spray suspension of mometasone and olopatadine may comprise one or more additional pharmaceutical active agent/s selected from the therapeutic category of, but not limited to, non-steroidal anti-inflammatory agents, decongestants, and any combination of any of the foregoing.

The aqueous nasal spray suspension can be administered as a drop or any other form suitable for topical administration. The composition may also be administered using a nasal tampon or a nasal sponge.

In a preferred embodiment, the aqueous suspension is provided in the form of nasal spray wherein the suspension is administered in a single unit-dose container or multi-dose container. Suitable single unit-dose containers or multi-dose containers include, but are not limited to, glass, aluminum, polypropylene or high density polyethylene, for example, high density polyethylene containers produced using a blow-fill-seal manufacturing technique.

In one embodiment, the pharmaceutical composition is contained within a spray bottle (such as a sprayer). The spray bottle may include a container for storing the pharmaceutical composition and a pump for spraying the pharmaceutical composition intranasally. In one embodiment, the container is capable of storing 30, 56, 60, or 120, or 240 doses. For example, a container storing 120 doses can provide a 1 month supply of the pharmaceutical composition (1 spray per nostril twice daily (4 sprays per day) for 30 days). A container storing 28 doses can provide a 1 week supply of the pharmaceutical composition (1 spray per nostril twice daily (4 sprays per day) for 7 days). In one embodiment, the container may be suitable for containing 4-5 mL (e.g., 4.5 mL or 28 doses), 9-10 mL of the pharmaceutical composition (e.g., 9 mL or 60 doses), or 18-19 mL (e.g., 18 mL or 120 doses) of the pharmaceutical composition.

In certain additional embodiments, the invention provides a multi dosage composition of matter, comprising: (a) a multi-unit dosage of a pharmaceutical composition of the present invention; and (b) a container comprising: (i) a squeezable chamber holding the multi dosage of the composition and having an opening wherein the dosage exits the opening when the squeezable chamber is squeezed; and (ii) a closure mechanism removably attached to the opening of the squeezable chamber. In certain embodiments, the multi dosage container is made of a moldable polymer.

In such embodiments, suitable polymers include, but are not limited to, polyethylene, polypropylene (PP), polystyrene (PS), nylon (Ny), polyvinyl chloride (PVC), polyethylene terephthalate (PET), polycarbonate (PC), polyoxymethylene (POM), polysulfon (PSF), polyethersulfon (PES), polyacrylate (PAR), and polyamid (PA). In certain embodiments, polymers include polyethylene, particularly medium-density polyethylene (MDPE) (or branched polyethylene) or high density polyethylene (HDPE) (or linear, polyethylene). In one embodiment, the multi dose container is made of high density polyethylene (HDPE).

Other means for delivering the nasal spray, such as inhalation via a metered dose inhaler (MDI), may also be used. Several types of MDIs are regularly used for administration by inhalation. These types of devices can include breath-actuated MDIs, spacer/holding chambers in combination with MDIs, and nebulizers. The term "MDI" as used herein refers to an inhalation delivery system comprising, for example, a canister containing a mixture of an active agent and a propellant optionally with one or more excipients, a metered dose valve, an actuator, and a mouthpiece. The canister is usually filled with a suspension of an active agent, such as the nasal spray composition, and a propellant, such as one or more hydrofluoroalkanes [e.g. 1, 1, 1, 2-tetrafluoroethane (HFA-134a) and 1, 1, 1, 2, 3, 3, 3-heptafluoropropane (HFA-227)], chlorofluorocarbons, and alcohols such as ethanol, isopropanol, butanol, propanol or mixtures thereof. When the actuator is depressed a metered dose of the suspension is aerosolized for inhalation. Particles comprising the active agent are propelled towards the mouthpiece where they may then be inhaled by a subject.

A further embodiment is a stable fixed dose, aqueous pharmaceutical composition (e.g., contained in a container) for nasal administration comprising about 0.025 % w/w to about 0.05 % w/w mometasone or its salt and about 0.5 % w/w to about 0.8 % w/w olopatadine or its salt for the treatment of rhinitis in a human in need thereof.

The following examples are provided to enable one skilled in the art to practice the invention and these are merely illustrative of the invention and should not be read as limiting the scope of the invention.

### EXAMPLES

### EXAMPLES 1 AND 2

Suspension compositions containing Mometasone furoate, Olopatadine HCl and Carboxymethylcellulose Sodium

| **SN** | **Ingredient** | **Example 1 (% w/w)** | **Example 2 (% w/w)** |
|---|---|---|---|
| 1 | Mometasone Furoate monohydrate | 0.050 | 0.025 |
| | Eq. to Mometasone furoate | | |
| 2 | Olopatadine Hydrochloride | 0.665 | 0.665 |
| 3 | Avicel RC 591 (Microcrystalline Cellulose and Carboxymethylcellulose Sodium) | 1.200 | 1.200 |
| 4 | Benzalkonium chloride (50 % solution) | 0.040 | 0.040 |
| 5 | Carboxymethylcellulose Sodium (Cekol 2000 P) | 0.500 | 0.500 |
| 6 | Sodium chloride | 0.410 | 0.410 |
| 7 | Edetate disodium | 0.010 | 0.010 |
| 8 | Dibasic sodium phosphate heptahydrate | 0.940 | 0.940 |
| 9 | Polysorbate 80 | 0.010 | 0.010 |
| 10 | Sodium Hydroxide | Q.S. | Q.S. |
| 11 | Hydrochloric acid | Q.S. | Q.S. |
| 12 | Water for injection | Q.S. | Q.S. |

| **Observations** | | | |
|---|---|---|---|
| | | | |
| | Physical observation on standing for 24 hours | No phase separation observed | No phase separation observed |
| | Mean Particle size by microscopy | Below 15 µm. | Below 15 µm. |

### Manufacturing procedure:

**1.** Avicel RC-591 was added in water for injection with homogenization and allowed to hydrate.
**2.** Carboxymethylcellulose Sodium was dispersed in water for injection and added to step -1.
**3.** Dibasic sodium phosphate heptahydrate, Sodium chloride, Edetate disodium and Olopatadine were dissolved in water. The pH was adjusted to 2.8 - 3.2 with Hydrochloric acid.
**4.** Step-3 was added to Step-1 with homogenization.
**5.** Polysorbate 80 was dissolved in water for injection. Mometasone Furoate monohydrate was added and stirred to form slurry.
**6.** Step-5 was added to Step-4 with homogenization.
**7.** Benzalkonium chloride was dissolved in water for injection.
**8.** Step-7 was added to Step-6 with homogenization.
**9.** The pH was checked and adjusted to 3.5-3.9 with HCl and the total weight was adjusted with Water for injection. The osmolality of the composition was about 250-350 mOsm/kg.

The composition was subjected to stability studies at different conditions. The results of the same are as follows:
Container details: Sprayer containing HDPE bottle crimped with pump and fitted with an actuator and cap.

| **Stability Study Data** | | | | | | |
|---|---|---|---|---|---|---|
| **Test** | | | | **Initial** | **3 months** | **6 months** |
| | **Ex. 1** | **Ex. 2** | **Ex. 1** | **Ex. 2** | **Ex. 1** | **Ex. 2** |
| **Stability condition** (25°C ± 2°C & 60% RH ± 5 % RH) | | | | | | |
| pH | 3.61 | | 3.69 | 3.73 | 3.78 | 3.81 |
| Osmolality (mOsm)* | 310 | 308 | 299 | 298 | 302 | 311 |
| Viscosity (cps)** | 32.5 | | 42.5 | 42.3 | 40.6 | 40.9 |
| Weight per ml (g/ml) | 1.01 | | 1.021 | 1.024 | 1.029 | 1.019 |
| Assay of mometasone furoate (% w/w) | 101 | 102.4 | 99.1 | 99.3 | 98.2 | 97.2 |
| Assay of olopatadine hydrochloride (% w/w) | 98.2 | 99.9 | 97.3 | 99.1 | 97.8 | 97.9 |

| Related substances for mometasone furoate | | | | | | |
|---|---|---|---|---|---|---|
| Impurity DMCF (%) | 0.02 | 0.03 | 0.09 | 0.10 | 0.14 | 0.17 |
| Any other impurity (%) | 0.04 | | 0.04 | | 0.03 | |
| Total impurities (%) | 0.09 | | 0.23 | 0.29 | 0.31 | 0.34 |

| Related substances for olopatadine hydrochloride | | | | | | |
|---|---|---|---|---|---|---|
| Olopatadine E-isomer (%) | 0.08 | | 0.07 | 0.09 | 0.09 | |
| Any other impurity (%) | 0.03 | 0.04 | 0.09 | 0.12 | 0.11 | 0.11 |
| Total impurities (%) | 0.15 | 0.16 | 0.20 | 0.25 | 0.37 | 0.38 |

| Spray Pattern (at 6cm) | | | | | | |
|---|---|---|---|---|---|---|
| Major Axis (mm) | 52 | | 60 | 63 | 59 | 61 |
| Minor Axis (mm) | 43 | 47 | 49 | 53 | 49 | 51 |
| Ellipticity | 1.2 | 1.1 | 1.2 | 1.2 | 1.2 | 1.2 |

| Droplet size distribution (at 6 cm) | | | | | | |
|---|---|---|---|---|---|---|
| D₁₀ (µm) | 18.91 | 19.45 | 19.26 | 19.70 | 19.33 | 18.88 |
| D₅₀ (µm) | 36.39 | 37.61 | 35.96 | 37.34 | 39.28 | 37.85 |
| D₉₀ (µm) | 72.46 | 76.44 | 70.29 | 75.78 | 85.42 | 72.07 |
| SPAN | 1.47 | 1.51 | 1.42 | 1.5 | 1.67 | 1.46 |

| **Stability condition** (40°C ± 2°C & 75% RH ± 5 % RH) | | | | | | |
|---|---|---|---|---|---|---|
| pH | 3.61 | | 3.68 | 3.72 | 3.59 | 3.68 |
| Osmolality (mOsm) | 310 | 308 | 298 | 306 | 305 | 299 |

| **Stability Study Data** | | | | | | |
|---|---|---|---|---|---|---|
| **Test** | **Initial** | | **3 months** | | **6 months** | |
| | **Ex. 1** | **Ex. 2** | **Ex. 1** | **Ex. 2** | **Ex. 1** | **Ex. 2** |
| Viscosity (cps) | 32.5 | | 45.2 | 42.6 | 41.8 | 41.5 |
| Weight per ml (g/ml) | 1.01 | | 1.023 | 1.019 | 1.026 | 1.025 |
| Assay of mometasone furoate (%) | 101 | 102.4 | 99.8 | 100.4 | 98.3 | 98.4 |
| Assay of oloptadine hydrochloride (%) | 98.2 | 99.9 | 99.3 | 102.5 | 98.7 | 99.7 |

| Related substances for mometasone furoate | | | | | | |
|---|---|---|---|---|---|---|
| Impurity DMCF (%) | 0.02 | 0.03 | 0.14 | 0.20 | 0.25 | 0.25 |
| Any other impurity (%) | 0.04 | 0.04 | 0.04 | 0.03 | 0.03 | 0.04 |
| Total impurities (%) | 0.09 | | 0.25 | 0.39 | 0.40 | 0.46 |

| Related substances for olopatadine hydrochloride | | | | | | |
|---|---|---|---|---|---|---|
| Olopatadine E-isomer (%) | 0.08 | | 0.07 | 0.08 | 0.08 | 0.09 |
| Any other impurity (%) | 0.03 | 0.04 | 0.21 | 0.18 | 0.31 | 0.30 |
| Total impurities (%) | 0.15 | 0.16 | 0.32 | 0.36 | 0.68 | 0.64 |

| Spray Pattern (at 6 cm) | | | | | | |
|---|---|---|---|---|---|---|
| Major Axis (mm) | 52 | 52 | 61 | 58 | 58 | 58 |
| Minor Axis (mm) | 43 | 47 | 50 | 49 | 48 | 49 |
| Ellipticity | 1.2 | 1.1 | 1.2 | 1.2 | 1.2 | 1.2 |

| Droplet size distribution (at 6 cm) | | | | | | |
|---|---|---|---|---|---|---|
| D₁₀ (µm) | 18.91 | 19.45 | 19.49 | 19.27 | 18.05 | 18.09 |
| D₅₀ (µm) | 36.39 | 37.61 | 35.29 | 34.68 | 36.19 | 36.12 |
| D₉₀ (µm) | 72.46 | 76.44 | 64.66 | 63.49 | 71.89 | 70.06 |
| SPAN | 1.47 | 1.51 | 1.28 | 1.27 | 1.50 | 1.44 |

| | | | | | | |
|---|---|---|---|---|---|---|
| * Determined by Advanced Instruments Osmometer (Model 3250). ** Determined by Brookfield viscometer. | | | | | | |

### EXAMPLES 3 AND 4

Suspension compositions containing Mometasone furoate, Olopatadine HCl and Xanthan Gum.

| **SN** | **Ingredient** | **Example 3** ( **% w/w)** | **Example 4 ( % w/w)** |
|---|---|---|---|
| 1 | Mometasone Furoate monohydrate | 0.050 | 0.025 |
| | Eq. to Mometasone furoate | | |
| 2 | Olopatadine HCl | 0.665 | 0.665 |
| 3 | Avicel RC 591 (Microcrystalline Cellulose and Carboxymethylcellulose Sodium) | 1.000 | 1.000 |
| 4 | Benzalkonium chloride (50 % solution) | 0.040 | 0.040 |
| 5 | Xantural 75 (Xanthan Gum) | 0.300 | 0.300 |
| 6 | Sodium chloride | 0.410 | 0.410 |
| 7 | Edetate disodium | 0.010 | 0.010 |
| 8 | Dibasic sodium phosphate heptahydrate | 0.940 | 0.940 |
| 9 | Polysorbate 80 | 0.010 | 0.010 |
| 10 | Sodium Hydroxide | Q.S. | Q.S. |
| 11 | Hydrochloric acid | Q.S. | Q.S. |
| 12 | Water for injection | Q.S. | Q.S. |

| **Observations** | | | |
|---|---|---|---|
| | Physical observation on standing for 24 hours | No phase separation observed | No phase separation observed |
| | Mean Particle size by microscopy | Below 15 µm. | Below 15 µm. |

### Manufacturing procedure:

**1.** Avicel RC-591 was added in Water for injection with homogenization and allowed to hydrate.
**2.** Xanthan gum was dispersed in Water for injection and added to step -1.
**3.** Dibasic sodium phosphate heptahydrate, Sodium chloride, Edetate disodium and Olopatadine were dissolved in water. The pH was adjusted to 2.8 -3.2 with Hydrochloric acid.
**4.** Step-3 was added to Step-1.
**5.** Polysorbate 80 was dissolved in water for injection. Mometasone Furoate monohydrate was added to it and stirred to form slurry.
**6.** Step-5 was added to Step-4 with homogenization.
**7.** Benzalkonium chloride was dissolved in water for injection.
**8.** Step-7 was added to Step-6 with homogenization.
**9.** The pH was checked and adjusted to 3.5-3.9 with HCl and the weight was adjusted with water for injection. The osmolality of the composition was about 250-350 mOsm/kg.

The composition was subjected to stability studies at different conditions. The results of the same are as follows:
Container details: Sprayer containing HDPE bottle crimped with pump and fitted with a actuator and cap

| **Stability Study Results** | | | | | | |
|---|---|---|---|---|---|---|
| **Test** | **Initial** | | **3 months** | | **6 months** | |
| | **Ex. 3** | **Ex. 4** | **Ex. 3** | **Ex. 4** | **Ex. 3** | **Ex. 4** |
| **Stability condition** (25°C ± 2°C & 60% RH ± 5 % RH) | | | | | | |
| pH | 3.65 | 3.67 | 3.78 | 3.65 | 3.70 | 3.62 |
| Osmolality (mOsm) | 307 | 312 | 302 | 316 | 308 | 308 |
| Viscosity (cps) | 124.2 | 129.1 | 127.9 | 129.9 | 126.2 | 126.8 |
| Weight per ml (g/ml) | 1.015 | 1.022 | 1.02 | 1.023 | 1.02 | 1.019 |
| Assay of mometasone furoate (%) | 99.9 | 102.8 | 102.2 | 99.0 | 98.7 | 100.4 |
| Assay of olopatadine hydrochloride (%) | 99.2 | 100.7 | 99.7 | 99.7 | 99.4 | 99.6 |

| Related substances for mometasone furoate | | | | | | |
|---|---|---|---|---|---|---|
| Impurity DMCF (%) | 0.02 | 0.02 | 0.04 | 0.05 | 0.03 | 0.05 |
| Any other impurity (%) | 0.03 | | 0.04 | | 0.03 | 0.04 |
| Total impurities (%) | 0.11 | 0.10 | 0.15 | 0.16 | 0.12 | 0.16 |

| Related substances for olopatadine hydrochloride | | | | | | |
|---|---|---|---|---|---|---|
| Olopatadine E-isomer (%) | 0.08 | 0.07 | 0.09 | 0.11 | 0.11 | 0.10 |
| Any other impurity (%) | 0.03 | 0.04 | 0.05 | 0.05 | 0.08 | 0.08 |
| Total impurities (%) | 0.18 | 0.15 | 0.24 | 0.20 | 0.33 | 0.33 |

| Spray Pattern (at 6cm) | | | | | | |
|---|---|---|---|---|---|---|
| Major Axis (mm) | 46 | | 59 | 59 | 56 | 54 |
| Minor Axis (mm) | 38 | | 47 | 44 | 35 | 43 |
| Ellipticity | 1.2 | | 1.3 | 1.4 | 1.6 | 1.3 |

| Droplet size distribution (at 6 cm) | | | | | | |
|---|---|---|---|---|---|---|
| D₁₀ (µm) | 21.58 | 21.03 | 20.95 | 20.27 | 18.73 | 18.34 |
| D₅₀ (µm) | 40.44 | 39.79 | 37.86 | 37.93 | 36.66 | 36.16 |
| D₉₀ (µm) | 78.25 | 77.55 | 74.07 | 74.93 | 70.63 | 70.99 |
| SPAN | 1.40 | 1.42 | 1.40 | 1.44 | 1.41 | 1.45 |

| **Stability condition** (40°C ± 2°C & 75% RH ± 5 % RH) | | | | | | |
|---|---|---|---|---|---|---|
| pH | 3.65 | 3.67 | 3.70 | 3.77 | 3.78 | 3.65 |
| Osmolality (mOsm) | 307 | 312 | 309 | 305 | 302 | 316 |
| Viscosity (cps) | 124.2 | 129.1 | 129.6 | 124.3 | 127.9 | 129.9 |
| Weight per ml (g/ml) | 1.015 | 1.022 | 1.017 | 1.027 | 1.022 | 1.020 |
| Assay of mometasone furoate (%) | 99.9 | 102.8 | 101.7 | 100.6 | 99.6 | 98.9 |
| Assay of oloptadine hydrochloride (%) | 99.2 | 100.7 | 99.9 | 99.4 | 99.7 | 99.9 |

| **Stability Study Results** | | | | | | |
|---|---|---|---|---|---|---|
| **Test** | **Initial** | | **3 months** | | **6 months** | |
| | **Ex. 3** | **Ex. 4** | **Ex. 3** | **Ex. 4** | **Ex. 3** | **Ex. 4** |
| Related substances for mometasone furoate | | | | | | |
| Impurity DMCF (%) | 0.02 | 0.02 | 0.10 | 0.12 | 0.10 | 0.12 |
| Any other impurity (%) | 0.03 | 0.03 | 0.02 | 0.03 | 0.05 | 0.03 |
| Total impurities (%) | 0.11 | 0.10 | 0.20 | 0.22 | 0.18 | 0.21 |

| Related substances for olopatadine hydrochloride | | | | | | |
|---|---|---|---|---|---|---|
| Olopatadine E-isomer (%) | 0.08 | 0.07 | 0.12 | 0.13 | 0.11 | 0.11 |
| Any other impurity (%) | 0.03 | 0.04 | 0.06 | 0.06 | 0.12 | 0.12 |
| Total impurities (%) | 0.18 | 0.15 | 0.26 | 0.26 | 0.41 | 0.40 |

| Spray Pattern (at 6cm) | | | | | | |
|---|---|---|---|---|---|---|
| Major Axis (mm) | 46 | 46 | 56 | 58 | 54 | 55 |
| Minor Axis (mm) | 38 | 38 | 45 | 49 | 34 | 43 |
| Ellipticity | 1.2 | 1.2 | 1.3 | 1.2 | 1.6 | 1.3 |

| Droplet size distribution (at 6 cm) | | | | | | |
|---|---|---|---|---|---|---|
| D₁₀ (µm) | 21.58 | 21.03 | 20.67 | 23.16 | 19.13 | 19.16 |
| D₅₀ (µm) | 40.44 | 39.79 | 38.06 | 39.08 | 37.34 | 37.26 |
| D₉₀ (µm) | 78.25 | 77.55 | 75.63 | 69.37 | 72.36 | 72.49 |
| SPAN | 1.40 | 1.42 | 1.44 | 1.19 | 1.42 | 1.43 |

### COMPARATIVE EXAMPLES A AND B

Suspension composition containing Mometasone furoate, and Olopatadine HCl.

| **SN** | **Ingredient** | **Example (% w/w)** | |
|---|---|---|---|
| | | **A** | **B** |
| 1 | Mometasone Furoate monohydrate | 0.050 | 0.050 |
| | Eq. to Mometasone furoate | | |
| 2 | Olopatadine HCl | 0.665 | 0.665 |
| 3 | Avicel RC 591 (Microcrystalline Cellulose and Carboxymethyl cellulose Sodium) | 1.00 | 1.00 |
| 4 | Benzalkonium chloride (50 % solution) | 0.040 | 0.040 |
| 5 | Carboxymethylcellulose Sodium (Cekol 2000 P) | 0.00 | 0.150 |
| 6 | Sodium chloride | 0.410 | 0.410 |
| 7 | Edetate disodium | 0.010 | 0.010 |
| 8 | Dibasic sodium phosphate heptahydrate | 0.940 | 0.940 |
| 9 | Polysorbate 80 | 0.010 | 0.010 |
| 10 | Sodium Hydroxide | Q.S. | Q.S. |
| 11 | Hydrochloric acid | Q.S. | Q.S. |
| 12 | Water for injection | Q.S. | Q.S. |

| **Observations** | | | |
|---|---|---|---|
| | pH | 3.7 | 3.7 |
| | Physical observation on standing for 24 hours | Phase separation observed | Phase separation observed |

### Manufacturing procedure:

The manufacturing procedure as mentioned in Example 1 was followed.

### COMPARATIVE EXAMPLES C AND D

Suspension composition containing Mometasone furoate and Olopatadine HCl.

| **SN** | **Ingredient** | **Example (% w/w)** | |
|---|---|---|---|
| | | **C** | **D** |
| 1 | Mometasone Furoate monohydrate | | |
| | Eq. to Mometasone furoate | 0.050 | 0.050 |
| 2 | Olopatadine HCl | 0.665 | 0.665 |
| 3 | Avicel RC 591 (Microcrystalline Cellulose and Carboxymethyl cellulose Sodium) | 1.000 | 1.000 |
| 4 | Benzalkonium chloride (50 % solution) | 0.040 | 0.040 |
| **5** | Xantural 75 (Xanthan Gum) | 0.00 | 0.20 |
| 6 | Sodium chloride | 0.410 | 0.410 |
| 7 | Edetate disodium | 0.010 | 0.010 |
| 8 | Dibasic sodium phosphate heptahydrate | 0.940 | 0.940 |
| 9 | Polysorbate 80 | 0.010 | 0.010 |
| 10 | Sodium Hydroxide | Q.S. | Q.S. |
| 11 | Hydrochloric acid | Q.S. | Q.S. |
| 12 | Water for injection | Q.S. | Q.S. |

| **Observations** | | | |
|---|---|---|---|
| | pH | 3.73 | 3.70 |
| | Physical observation on standing for 24 hours | Phase separation observed | Phase separation observed |

### Manufacturing procedure:

The manufacturing procedure as mentioned in Example 3 was followed.

### EXAMPLE 5

### Phase III Clinical Study of Fixed Dose Combination of Mometasone and Olopatadine Nasal Spray in Pediatric Patients

This study is a double blind, randomized, parallel-group, 12-week study to evaluate the efficacy, safety, and tolerability of a fixed dose combination of mometasone furoate and olopatadine hydrochloride nasal spray compared with a placebo nasal spray in pediatric subjects (aged 2 to under 12 years) with perennial allergic rhinitis (PAR).

### Study Objectives

To compare the efficacy of mometasone furoate and olopatadine hydrochloride nasal spray (administered as 1 spray per nostril twice daily) with a placebo nasal spray for treatment in pediatric subjects (aged ≥ 2 to < 12 years) with PAR.

A secondary objective is to compare the safety and tolerability of mometasone furoate and olopatadine hydrochloride nasal spray with a placebo nasal spray over 12 weeks of study treatment.

### Key Subject Selection Criteria

- Male or non-pregnant female subjects aged ≥ 2 to < 12 years, as of the Screening Visit (Visit 1).
- Documented clinical history of PAR (≥ 12 months for subjects aged ≥ 6 to < 12 years, ≥ 6 months for subjects aged ≥ 2 to < 6 years preceding the Screening Visit [Visit 1]) with exacerbations (clinical evidence of active symptoms). In the judgment of the Investigator, the PAR must have been of sufficient severity to have required treatment (either continuous or intermittent) in the past and is expected to require treatment for the study duration.
- Documented positive skin prick test (wheal diameter at least 3 mm greater than negative control wheal) to at least one allergen known to induce PAR. Documentation of a positive result within 12 months prior to the Screening Visit (Visit 1) is acceptable. Positive allergen test for the subject that must be consistent with the medical history of PAR. Additionally the subject is expected to be exposed to the PAR allergen that he or she tested positive for via the skin prick test for the entire duration of the study.
- A 12-hour rTNSS value of ≥ 6 (out of a possible 12) for the AM assessment at the Screening Visit (Visit 1).

### Study Design

A total of approximately 540 subjects (≥ 2 to <12 years) will be randomized in the study in a 2:1 ratio for a fixed dose combination of mometasone furoate and olopatadine hydrochloride nasal spray (360 subjects) versus placebo nasal spray (180 subjects).

The subject participation may be 22 days up to 27 days with 7 to 10 days of a screening/run-in period and 14 days of treatment period, with allowable window periods for the study visits. The treatment groups are provided in the table below.

### Investigational products and their administration

| **Investigational product(s)** | **Administration** |
|---|---|
| Olopatadine hydrochloride + mometasone furoate (665 µg + 25 µg) nasal spray* [Fixed Dose Combination] | 1 spray per nostril, twice daily (BID) in morning and evening |
| Placebo nasal spray | Placebo - 1 spray per nostril twice daily (BID) in morning and evening |

| | |
|---|---|
| * - Each spray provides 665 µg olopatadine hydrochloride and 25 µg mometasone furoate. | |

### Primary Endpoint:

- Change from baseline in average AM and PM subject-reported 12-hour reflective Total Nasal Symptom Score (rTNSS) over the first 4 weeks of treatment for subjects aged ≥6 to <12 years.

### Secondary Endpoint(s):

- Change from baseline in average AM and PM subject-reported 12-hour instantaneous Total Nasal Symptom Score (iTNSS) over the first 4 weeks of treatment for subjects ≥6 to <12 years of age.
- Change from baseline in average AM and PM subject-reported 12-hour rTNSS over the first 4 weeks of treatment for subjects ≥2 to <12 years of age.
- Change from baseline in average AM and PM subject-reported 12-hour iTNSS over the first 4 weeks of treatment for subjects ≥2 to <12 years of age.
- Change from baseline in the overall Pediatric Rhinoconjunctivitis Quality of Life Questionnaire (PRQLQ) score at Week 4 between treatment groups.

### Other Endpoint(s):

### Nasal symptoms:

### TNSS - first 4 weeks, subjects aged ≥ 6 to ≤ 12 years:

- Change from baseline in AM subject-reported rTNSS over the first 4 weeks of treatment.
- Change from baseline in PM subject-reported rTNSS over the first 4 weeks of treatment.
- Change from baseline in AM subject-reported iTNSS over the first 4 weeks of treatment.
- Change from baseline in PM subject-reported iTNSS over the first 4 weeks of treatment.
- Change from baseline in subject-reported reflective individual nasal symptoms over the first 4 weeks of treatment period (AM, PM and average of AM and PM).
- Change from baseline in subject-reported instantaneous individual nasal symptoms over the first 4 weeks of treatment period (AM, PM and average of AM and PM).
- Change from baseline subject-reported rTNSS and iTNSS for each day (AM, PM and average of AM and PM).

### TNSS - first 4 weeks, subjects aged ≥ 2 to ≤ 6 years:

- Change from baseline in average AM and PM subject-reported 12-hour rTNSS over the first 4 weeks of treatment for subjects aged ≥2 to <6 years.
- Change from baseline in average AM and PM subject-reported 12-hour iTNSS over the first 4 weeks of treatment for subjects aged ≥2 to <6 years.

### TNSS - first 4 weeks, subjects aged ≥2 to <12 years:

- Change from baseline in average AM and PM subject-reported 12-hour rTNSS over the first 4 weeks of treatment for subjects aged ≥2 to <12 years.
- Change from baseline in average AM and PM subject-reported 12-hour iTNSS over the first 4 weeks of treatment for subjects aged ≥2 to <12 years.

Additional Total Nasal Symptom Score (TNSS) outcomes will be assessed for the following (e.g. AM, PM, Individual symptoms):
- 12 weeks, subjects aged ≥6 to <12 years.
- 12 weeks, subjects aged ≥2 to <6 years.
- 12 weeks, subjects aged ≥2 to <12 years.

### Physician Assessed Nasal Symptom Score (PNSS):

- Change from baseline in PNSS and physician assessed individual nasal symptoms at Weeks 4 and 12.

### Pediatric Rhinoconjuntivitis Quality of Life Questionnaire (PRQLQ):

- Individual domains of the PRQLQ at Weeks 4 and 12.

### EXAMPLE 6

### Phase III Clinical Study of Fixed Dose Combination of Mometasone and Olopatadine Nasal Spray in Pediatric Patients

This study was a double blind, randomized, parallel-group 14-day study to evaluate the efficacy, safety, and tolerability of a fixed dose combination of mometasone furoate and olopatadine hydrochloride nasal spray compared with a placebo nasal spray in pediatric subjects (aged 6 to under 12 years) with seasonal allergic rhinitis (SAR).

### Study Objectives

To compare the efficacy of mometasone furoate and olopatadine hydrochloride nasal spray (administered as 1 spray per nostril twice daily) with a placebo nasal spray for treatment in pediatric subjects (aged ≥ 6 to <12 years) with SAR.

A secondary objective was to compare the safety and tolerability of mometasone furoate and olopatadine hydrochloride nasal spray with a placebo nasal spray over the study period.

### Key Subject Selection Criteria

- Male or non-pregnant female subjects aged ≥ 6 to < 12 years, at the Screening Visit (Visit 1).
- Documented clinical history of SAR (for at least 2 years preceding the Screening Visit [Visit 1]) with exacerbations (clinical evidence of active symptoms) during the study season for the relevant seasonal allergen (tree/grass pollen). SAR must have been of sufficient severity to have required treatment (either continuous or intermittent) in the past, and in the Investigator's judgment, is expected to require treatment throughout the study period.
- Demonstrated sensitivity to at least 1 seasonal allergen (tree/grass pollen) known to induce SAR through a documented positive skin prick test (wheal diameter at least 5 mm greater than the negative control) to a relevant seasonal allergen. Documentation of a positive result within 12 months prior to the Screening Visit (Visit 1) is acceptable. The subject's positive allergen must be consistent with the medical history of SAR. Additionally, the subject is expected to be adequately exposed to the SAR allergen that he/she has tested positive for the entire duration of the study.
- A 12-hour reflective Total Nasal Symptom Score (rTNSS) value of ≥ 6 (out of a possible 12) for the morning (AM) assessment at the Screening Visit (Visit 1).

### Study Design

A total of 446 subjects (≥ 2 to <12 years) were randomized in the study in a 1:1 ratio for a fixed dose combination of mometasone furoate and olopatadine hydrochloride nasal spray (225 subjects) versus placebo nasal spray (225 subjects).

The treatment groups are provided in the table below.

### Investigational products and their administration

| **Investigational product(s)** | **Administration** |
|---|---|
| Olopatadine hydrochloride + mometasone furoate (665 µg + 25 µg) [Fixed Dose Combination] nasal spray* | 1 spray per nostril, twice daily (BID) in morning and evening |
| Placebo nasal spray | Placebo - 1 spray per nostril twice daily (BID) in morning and evening |

| | |
|---|---|
| * - Each spray provides 665 µg olopatadine hydrochloride and 25 µg mometasone furoate. | |

The study was 12 weeks in duration consisting of up to 7 to 10 days of a screening/placebo run-in period and 12 weeks of treatment period with allowable window periods for the study visits.

### Key evaluation Criteria (Clinical Endpoints):

### Primary Endpoint

- Change from baseline in average AM and PM subject-reported 12-hour reflective Total Nasal Symptom Score (rTNSS) over the 14-day treatment period.

### Secondary Endpoint(s):

- Change from baseline in average AM and PM subject-reported 12-hour instantaneous Total Nasal Symptom Score (iTNSS) over the 14-day treatment period.
- Change from baseline in the overall Pediatric Rhinoconjunctivitis Quality of Life Questionnaire (PRQLQ) score on Day 15 (Visit 4) between treatment groups.
- Change from baseline in average AM and PM subject-reported 12-hour reflective Total Ocular Symptom Score (rTOSS) over the 14-day treatment period.

### Other Efficacy Endpoint(s):

### Nasal Symptoms

- Change from baseline in AM subject-reported rTNSS over the 14-day treatment period.
- Change from baseline in AM subject-reported iTNSS over the 14-day treatment period.
- Change from baseline in PM subject-reported rTNSS over the 14-day treatment period.
- Change from baseline in PM subject-reported iTNSS over the 14-day treatment period.
- Change from baseline in subject-reported reflective individual nasal symptoms over the 14-day treatment period (AM, PM and average of AM and PM).
- Change from baseline in subject-reported instantaneous individual nasal symptoms over the 14-day treatment period (AM, PM and average of AM and PM).
- Change from baseline in average AM and PM subject-reported rTNSS and iTNSS for each day.
- Change from baseline in AM subject-reported rTNSS and iTNSS for each day.
- Change from baseline in PM subject-reported rTNSS and iTNSS for each day.

### Ocular Symptoms:

- Change from baseline in average AM and PM subject-reported instantaneous Total Ocular Symptom Score (iTOSS) over the 14-day treatment period.
- Change from baseline in AM subject-reported rTOSS over the 14-day treatment period.
- Change from baseline in AM subject-reported iTOSS over the 14-day treatment period.
- Change from baseline in PM subject-reported rTOSS over the 14-day treatment period.
- Change from baseline in PM subject-reported iTOSS over the 14-day treatment period.
- Change from baseline in subject-reported reflective individual ocular symptoms over the 14-day treatment period (AM, PM, and average AM and PM).
- Change from baseline in subject-reported instantaneous individual ocular symptoms over the 14-day treatment period (AM, PM, and average AM and PM).
- Change from baseline in average of the AM and PM subject-reported rTOSS and iTOSS for each day.
- Change from baseline in AM subject-reported rTOSS and iTOSS for each day.
- Change from baseline in PM subject-reported rTOSS and iTOSS for each day.

Non-nasal symptoms were assessed in a similar manner to the ocular symptoms above (as described in the Statistical Analysis Plan [SAP]).

### Physician Assessed Nasal Symptom Score (PNSS):

- Change from baseline in PNSS and physician assessed individual nasal symptoms at Day 15 (Visit 4).

### Pediatric Rhinoconjuntivitis Quality of Life Questionnaire (PRQLQ):

- Change from baseline in individual domains of the PRQLQ at Day 15.

The table below shows a summary of the primary clinical endpoint (average AM and PM rTNSS) and secondary clinical endpoints (average AM and PM iTNSS, average AM and PM rTOSS, and PRQLQ [Pediatric Rhinoconjunctivitis Quality of Life Questionnaire]) observed during this phase 3 study. For a comparison of the Fixed Dose Combination nasal spray versus a placebo nasal spray, a p-value below 0.05 is considered statistically significant.

### Summary of Primary and Secondary Clinical Endpoints

| | Primary Clinical Endpoint | Secondary Clinical Endpoint Least Square Mean Difference (95% | | |
|---|---|---|---|---|
| Treatment Arm | rTNSS (Least Square Mean Difference [p value]) | iTNSS (Least Square Mean Difference [p value]) | rTOSS (Least Square Mean Difference [p value]) | PRQLQ (Least Square Mean Difference [p value]) |
| Fixed Dose Combination Nasal Spray | -0.6 (p=0.001)* | -0.6 (p<0.001)* | -0.2 (p=0.233) | -0.3 (p<0.001)* |
| versus | | | | |
| Placebo Nasal Spray | | | | |

| | | | | |
|---|---|---|---|---|
| *indicates statistically significant iTNSS = instantaneous Total Nasal Symptom Score; rTNSS = reflective Total Nasal Symptom Score; rTOSS = reflective Total Ocular Symptom Score; PRQLQ = Pediatric Rhinoconjunctivitis Quality of Life Questionnaire | | | | |

The above table shows that the Fixed Dose Combination nasal spray (1 spray per nostril) when administered twice daily, is statistically superior to the placebo nasal spray (p<0.05) for the primary endpoint, change in average AM and PM rTNSS from baseline, and secondary endpoints, change in average AM and PM iTNSS from baseline and overall PRQLQ score.

The table below shows the least square mean difference in individual reflective and instantaneous nasal symptoms scores and individual domains of PRQLQ for the Fixed Dose Combination group compared to the placebo nasal spray group.

### Summary of Average AM and PM Reflective and Instantaneous Individual Nasal Symptoms and Individual Domains of PRQLQ Over the 14-Day Treatment

| Least Squares Mean Difference in Average AM and PM Reflective and Instantaneous Individual Nasal Symptom Scores with Fixed Dose Combination Nasal Spray Versus Placebo Nasal Spray | | |
|---|---|---|
| | Least Square Mean Difference (95% Confidence Interval) | *P* value |
| **Reflective** | | |
| Rhinorrhea | -0.1 (-0.2, 0.0) | 0.016* |
| Nasal Congestion | -0.1 (-0.2, 0.0) | 0.048* |
| Nasal Itching | -0.2 (-0.3, -0.1) | 0.002* |
| Sneezing | -0.2 (-0.3, -0.1) | <0.001* |

| **Instantaneous** | | |
|---|---|---|
| Rhinorrhea | -0.1 (-0.2, 0.0) | 0.003 * |
| Nasal Congestion | -0.1 (-0.2, -0.1) | 0.002* |
| Nasal Itching | -0.1 (-0.2, 0.1) | 0.408 |
| Sneezing | -0.2 (-0.3, -0.1) | <0.001* |

| Least Squares Mean Difference in Individual Domains of PRQLQ with Fixed Dose Combination Nasal Spray Versus Placebo Nasal Spray | | |
|---|---|---|
| Activity Limitations | -0.2 (-0.5, 0.0) | 0.043* |
| Practical Problems | -0.3 (-0.4, -0.1) | 0.006* |
| Nose Symptoms | -0.6 (-0.9, -0.4) | <0.001* |
| Eye Symptoms | -0.1 (-0.3, 0.1) | 0.203 |
| Other Symptoms | -0.2 (-0.4, 0.0) | 0.025* |

| | | |
|---|---|---|
| *indicates statistically significant | | |

As can be seen from the above table, treatment with the Fixed Dose Combination nasal spray exhibited significant difference (p < 0.05) compared to placebo nasal spray in average AM and PM rTNSS starting at Day 3 through Day 14. The Fixed Dose Combination nasal spray showed significant difference (p < 0.05) when compared to placebo nasal spray in average AM and PM iTNSS starting at Day 1 through Day 14.

Additionally, AM rTNSS, PM rTNSS, AM iTNSS, and PM iTNSS were analyzed overall and each day from Day 1 to Day 15. The Fixed Dose Combination nasal spray was statistically superior versus the placebo nasal spray for the change in average AM rTNSS, average PM rTNSS, average AM iTNSS, and average PM iTNSS. The results also showed statistically significant difference (p < 0.05) in favor of the Fixed Dose Combination nasal spray starting at Day 4 through Day 15 for both AM rTNSS and PM rTNSS. The results were also statistically significant (p < 0.05) for AM iTNSS and PM iTNSS in favor of the Fixed Dose Combination nasal spray starting at Day 1 through Day 15, with the exception of p value of 0.55 for PM iTNSS on Day 2.

The table below shows the least square mean difference in average AM and average PM individual reflective and instantaneous nasal symptoms scores for the Fixed Dose Combination group versus the placebo nasal spray group.

### Summary of Average AM and Average PM Reflective and Instantaneous Individual Nasal Symptoms Score Over the 14-Day Treatment

| Least Squares Mean Difference in Average AM and Average PM Reflective and Instantaneous Individual Nasal Symptoms Score With Fixed Dose Combination Nasal Spray versus Placebo Nasal spray | | |
|---|---|---|
| | Least Square Mean Difference (95% Confidence Interval) | *P* value |

| **AM Reflective** | | |
|---|---|---|
| Overall | -0.7 (-1.0, -0.3) | <0.001 * |
| Rhinorrhea | -0.2 (-0.3, 0.0) | 0.006* |
| Nasal Congestion | -0.1 (-0.2, 0.0) | 0.038* |
| Nasal Itching | -0.2 (-0.3, -0.1) | 0.003 * |
| Sneezing | -0.3 (-0.4, -0.2) | <0.001 * |

| **PM Reflective** | | |
|---|---|---|
| Overall | -0.5 (-0.9, -0.2) | 0.003* |
| Rhinorrhea | -0.1 (-0.2, 0.0) | 0.015* |
| Nasal Congestion | -0.1 (-0.2, 0.0) | 0.057 |
| Nasal Itching | -0.2 (-0.3, -0.1) | 0.002* |
| Sneezing | -0.2 (-0.3, -0.1) | <0.001* |

| **Instantaneous** | | |
|---|---|---|
| **AM Instantaneous** | | |
| Overall | -0.7 (-1.0, -0.3) | <0.001 * |
| Rhinorrhea | -0.2 (-0.3, -0.1) | 0.002* |
| Nasal Congestion | -0.2 (-0.3, -0.1) | <0.001* |
| Nasal Itching | -0.1 (-0.2, 0.0) | 0.014* |
| Sneezing | -0.2 (-0.3, -0.1) | <0.001* |

| **PM Instantaneous** | | |
|---|---|---|
| Overall | -0.7 (-1.1, -0.4) | <0.001 * |
| Rhinorrhea | -0.2 (-0.3, -0.1) | 0.002* |
| Nasal Congestion | -0.2 (-0.3, -0.1) | <0.001* |
| Nasal Itching | -0.1 (-0.2, 0.1) | 0.293 |
| Sneezing | -0.3 (-0.4, -0.2) | <0.001* |

| | | |
|---|---|---|
| *indicates statistically significant | | |

The table below summarizes the results of overall physician assessment of nasal symptoms score (PNSS) and individual nasal symptom assessment (rhinorrhea, nasal congestion, nasal itching, and sneezing).

### Summary of Average PNSS and Individual Domains of PNSS Over the 14-Day Treatment

| Least Squares Mean Difference in Average PNSS and Individual Domains of PNSS With Fixed Dose Combination NS Versus Placebo NS | | |
|---|---|---|
| | Least Square Mean Difference (95% Confidence Interval) | *P* value |
| | | |
| Overall PNSS | -0.9 (-1.5, -0.2) | 0.01* |
| Rhinorrhea | -0.3 (-0.4, -0.1) | <0.001 * |
| Nasal Congestion | -0.2 (-0.5, 0.0) | 0.022* |
| Nasal Itching | -0.3 (-0.4, -0.1) | <0.001 * |
| Sneezing | -0.2 (-0.4, -0.1) | <0.001 * |

| | | |
|---|---|---|
| *indicates statistically significant | | |

The safety profile of the Fixed Dose Combination nasal spray was well-tolerated and found to be similar to the placebo nasal spray in subjects aged 6 to < 12 years. Out of a total of 446 randomized subjects, 27 (12.0%) subjects experienced at least 1 TEAE in the Fixed Dose Combination nasal spray group and 23 (10.4%) subjects experienced at least 1 TEAE in the placebo nasal spray group. No deaths were reported and only 1 SAE in the placebo nasal spary group was reported. All TEAEs except one SAE were mild or moderate in severity. The most frequently reported TEAEs that were related to the study treatment (> 1% of subjects in any treatment group) were dysgeusia, headache, epistaxis, and ENT examination abnormal. No clinically significant differences in vital signs, physical exams, or focused ENT exams were observed.

Although the invention herein has been described with reference to particular embodiments, it is to be understood that these embodiments are merely illustrative of the principles and application of the present invention. It is therefore to be understood that numerous modifications may be made to the illustrative embodiments.

## Claims

1. A fixed-dose pharmaceutical composition comprising mometasone furoate and olopatadine hydrochloride for use in treating allergic rhinitis in a pediatric human subject in need thereof, wherein (i) the composition is nasally administered as one spray per nostril of the pediatric human subject twice daily, (ii) each spray comprises about 25 mcg of mometasone furoate and about 665 mcg olopatadine hydrochloride, and (iii) the administration provides relief from one or more symptoms of allergic rhinitis faster than nasal administration of 25 mcg mometasone furoate or 665 mcg olopatadine hydrochloride alone; wherein the pediatric human subject is 2 to 11 years of age.

2. The composition of claim 1, wherein the symptoms are nasal symptoms.

3. The composition of claim 1, wherein the symptoms are selected from nasal congestion, rhinorrhea, itching and sneezing.

4. The composition of claim 1, wherein the allergic rhinitis is seasonal allergic rhinitis.

5. The composition of claim 1, wherein the allergic rhinitis is perennial allergic rhinitis.

6. The composition of any one of claims 1-5, wherein administration of the pharmaceutical composition provides relief from one or more symptoms of allergic rhinitis within 15 minutes of administration.

7. A pharmaceutical composition comprising mometasone furoate and olopatadine hydrochloride for use in treating allergic rhinitis in a pediatric human subject in need thereof, wherein (i) the composition is nasally administered as one spray per nostril of the pediatric human subject twice daily, (ii) the pharmaceutical composition provides an onset of action within 15 minutes for the treatment of allergic rhinitis and (iii) each spray of the pharmaceutical composition comprises about 25 mcg of mometasone furoate and about 665 mcg of olopatadine hydrochloride; wherein the pediatric human subject is 2 to 11 years of age.

8. The composition of claim 7, wherein the symptoms are nasal symptoms.

9. The composition of claim 7, wherein the symptoms are selected from nasal congestion, rhinorrhea, itching and sneezing.

10. The composition of claim 7, wherein the allergic rhinitis is seasonal allergic rhinitis.

11. The composition of claim 7, wherein the allergic rhinitis is perennial allergic rhinitis.

## Patentansprüche

1. Pharmazeutische Zusammensetzung mit fester Dosis, umfassend Mometasonfuroat und Olopatadinhydrochlorid, zur Verwendung beim Behandeln von allergischer Rhinitis bei einem pädiatrischen menschlichen Subjekt, das dessen bedarf, wobei (i) die Zusammensetzung zweimal täglich als ein Sprühstoß pro Nasenloch des pädiatrischen menschlichen Subjekts nasal verabreicht wird, (ii) jeder Sprühstoß etwa 25 µg Mometasonfuroat und etwa 665 µg Olopatadinhydrochlorid umfasst und (iii) die Verabreichung schneller Linderung von einem oder mehreren Symptomen von allergischer Rhinitis bereitstellt als die nasale Verabreichung von 25 µg Mometasonfuroat oder 665 µg Olopatadinhydrochlorid allein; wobei das pädiatrische menschliche Subjekt 2 bis 11 Jahre alt ist.

2. Zusammensetzung nach Anspruch 1, wobei die Symptome nasale Symptome sind.

3. Zusammensetzung nach Anspruch 1, wobei die Symptome aus nasaler Kongestion, Rhinorrhoe, Juckreiz und Niesen ausgewählt sind.

4. Zusammensetzung nach Anspruch 1, wobei es sich bei der allergischen Rhinitis um saisonale allergische Rhinitis handelt.

5. Zusammensetzung nach Anspruch 1, wobei es sich bei der allergischen Rhinitis um ganzjährige allergische Rhinitis handelt.

6. Zusammensetzung nach einem der Ansprüche 1-5, wobei die Verabreichung der pharmazeutischen Zusammensetzung innerhalb von 15 Minuten nach der Verabreichung Linderung von einem oder mehreren Symptomen von allergischer Rhinitis bereitstellt.

7. Pharmazeutische Zusammensetzung, umfassend Mometasonfuroat und Olopatadinhydrochlorid, zur Verwendung beim Behandeln von allergischer Rhinitis bei einem pädiatrischen menschlichen Subjekt, das dessen bedarf, wobei (i) die Zusammensetzung zweimal täglich als ein Sprühstoß pro Nasenloch des pädiatrischen menschlichen Subjekts nasal verabreicht wird, (ii) die pharmazeutische Zusammensetzung einen Wirkungseintritt innerhalb von 15 Minuten zur Behandlung von allergischer Rhinitis bereitstellt und (iii) jeder Sprühstoß der pharmazeutischen Zusammensetzung etwa 25 µg Mometasonfuroat und etwa 665 µg Olopatadinhydrochlorid umfasst; wobei das pädiatrische menschliche Subjekt 2 bis 11 Jahre alt ist.

8. Zusammensetzung nach Anspruch 7, wobei die Symptome nasale Symptome sind.

9. Zusammensetzung nach Anspruch 7, wobei die Symptome aus nasaler Kongestion, Rhinorrhoe, Juckreiz und Niesen ausgewählt sind.

10. Zusammensetzung nach Anspruch 7, wobei es sich bei der allergischen Rhinitis um saisonale allergische Rhinitis handelt.

11. Zusammensetzung nach Anspruch 7, wobei es sich bei der allergischen Rhinitis um ganzjährige allergische Rhinitis handelt.

## Revendications

1. Composition pharmaceutique à dose fixe comprenant du furoate de mométasone et du chlorhydrate d'olopatadine destinée à être utilisée dans le traitement de la rhinite allergique chez un sujet humain pédiatrique en ayant besoin, dans laquelle (i) la composition est administrée par voie nasale sous forme d'une pulvérisation par narine du sujet humain pédiatrique deux fois par jour, (ii) chaque pulvérisation comprend environ 25 mcg de furoate de mométasone et environ 665 mcg de chlorhydrate d'olopatadine, et (iii) l'administration procure un soulagement d'un ou plusieurs symptômes de la rhinite allergique plus rapidement que l'administration nasale de 25 mcg de furoate de mométasone ou de 665 mcg de chlorhydrate d'olopatadine seuls ; dans laquelle le sujet humain pédiatrique est âgé de 2 à 11 ans.

2. Composition de la revendication 1, dans laquelle les symptômes sont des symptômes nasaux.

3. Composition de la revendication 1, dans laquelle les symptômes sont choisis parmi la congestion nasale, la rhinorrhée, les démangeaisons et les éternuements.

4. Composition de la revendication 1, dans laquelle la rhinite allergique est une rhinite allergique saisonnière.

5. Composition de la revendication 1, dans laquelle la rhinite allergique est une rhinite allergique perannuelle.

6. Composition de l'une quelconque des revendications 1 à 5, dans laquelle l'administration de la composition pharmaceutique procure un soulagement d'un ou de plusieurs symptômes de la rhinite allergique dans les 15 minutes suivant l'administration.

7. Composition pharmaceutique comprenant du furoate de mométasone et du chlorhydrate d'olopatadine destinée à être utilisée dans le traitement de la rhinite allergique chez un sujet humain pédiatrique en ayant besoin, dans laquelle (i) la composition est administrée par voie nasale sous forme d'une pulvérisation par narine du sujet humain pédiatrique deux fois par jour, (ii) la composition pharmaceutique fournit un début d'action en 15 minutes pour le traitement de la rhinite allergique et (iii) chaque pulvérisation de la composition pharmaceutique comprend environ 25 mcg de furoate de mométasone et environ 665 mcg de chlorhydrate d'olopatadine ; dans laquelle le sujet humain pédiatrique est âgé de 2 à 11 ans.

8. Composition de la revendication 7, dans laquelle les symptômes sont des symptômes nasaux.

9. Composition de la revendication 7, dans laquelle les symptômes sont choisis parmi la congestion nasale, la rhinorrhée, les démangeaisons et les éternuements.

10. Composition de la revendication 7, dans laquelle la rhinite allergique est une rhinite allergique saisonnière.

11. Composition de la revendication 7, dans laquelle la rhinite allergique est une rhinite allergique perannuelle.
